# EUROPÄISCHE PATENTSCHRIFT

(11) **EP 4 450 024 B1**
(45) Veröffentlichungstag und Bekanntmachung des Hinweises auf die Patenterteilung: **05.11.2025**
(21) Anmeldenummer: 24170206.7
(22) Anmeldetag: 15.04.2024
(51) Int. Cl.: A61C 13/15

(54) **STRAHLUNGSVORRICHTUNG ZUR AKTIVIERUNG POLYMERISIERBARER DENTALMATERIALIEN**
RADIATION DEVICE FOR ACTIVATING POLYMERISABLE DENTAL MATERIALS
DISPOSITIF DE RAYONNEMENT POUR L'ACTIVATION DE MATÉRIAUX DENTAIRES POLYMÉRISABLES

(30) Priorität: 18.04.2023 DE 102023109682
(43) Veröffentlichungstag der Anmeldung: 23.10.2024
(73) Patentinhaber: Kulzer GmbH, 63450 Hanau (DE)
(72) Erfinder: Utterodt, Andreas, 61267 Neu Anspach (DE)
(74) Vertreter: Pelster Behrends Patentanwälte PartG mbB

(56) Entgegenhaltungen:
- EP-B1- 3 122 229
- EP-B1- 3 442 464
- US-A1- 2022 273 399

## Beschreibung

Die Erfindung betrifft eine Strahlungsvorrichtung für den Einsatz zur Aktivierung von polymerisierbaren Dentalmaterialien und ein nicht-therapeutisches Verfahren zum Betrieb einer solchen Strahlungsvorrichtung. Offenbart wird zudem die Verwendung einer entsprechenden Strahlungsvorrichtung zur Aktivierung von polymerisierbaren Dentalmaterialien bei der Herstellung und/oder Bearbeitung dentaler Restaurationen.

In der modernen Zahnmedizin kommen heutzutage für die Herstellung dentaler Restaurationen, beispielsweise von Zahnfüllungen, regelmäßig kunststoffbasierte Dentalmaterialien zum Einsatz. Diese Dentalmaterialien sind polymerisierbar, insbesondere durch Lichtaktivierung polymerisierbar, und haben in dem für die Applikation vorgesehenen Zustand entsprechend noch nicht ihren finalen Aushärtungsgrad erreicht. Entsprechende Dentalmaterialien können vor der Aushärtung beispielsweise in eine in einem Zahn erzeugte Kavität eingegeben werden, wo sie sich optimal an die Form der Kavität anpassen lassen, sodass eine zuverlässige Reparatur des Defektes ermöglicht wird. Die entsprechenden Dentalmaterialien werden erst nach der Applikation ausgehärtet, was bedeutet, dass eine Vernetzung zwischen den im Dentalmaterial enthaltenen Komponenten ausgelöst wird, wodurch eine Aushärtung und damit eine Veränderung wesentlicher physikalisch-chemischer Parameter, beispielsweise der Härte und der Festigkeit, erreicht wird.

Auch wenn im Bereich der Polymerchemie prinzipiell verschiedene Mechanismen für die Aushärtung solcher polymerisierbaren Kunststoffe bekannt sind, beispielsweise durch thermische Aktivierung oder den Einsatz einer separaten Härterkomponente, kommt im dentalen Bereich insbesondere der strahlungsbasierten Härtung eine besondere Bedeutung zu, da diese für den angestrebten Einsatz im Mund eines Patienten mit erheblichen anwendungstechnischen Vorteilen verbunden ist.

Entsprechende strahlungshärtbare Dentalmaterialien, in denen die Polymerisation beispielsweise durch die Aktivierung mit blauem oder ultravioletten Licht initiiert werden kann, sind kommerziell von zahlreichen Herstellern erhältlich. In entsprechenden strahlungshärtbaren Dentalmaterialien werden dabei geeignete Photoinitiatoren eingesetzt, welche bei Bestrahlung mit elektromagnetischer Strahlung einer bestimmten Wellenlänge die Polymerisation des Dentalmaterials bedingen, beispielsweise indem der Zerfall des Photoinitiators radikalische Bestandteile bildet, welche zu einer radikalischen Polymerisation von ungesättigten Verbindungen im Dentalmaterial, insbesondere (Meth)acrylatverbindungen, führen können.

Zur Gewährleistung einer ausreichenden Vernetzung des eingebrachten Materials, insbesondere auch über die gesamte Tiefe der ausgefüllten Kavität hinweg, sowie zur Sicherstellung einer guten Reproduzierbarkeit, werden hohe Anforderungen an die von der Strahlungsvorrichtung emittierte Lichtintensität gestellt, die zur Erreichung einer optimalen Polymerisationsqualität insbesondere idealerweise auch auf das eingesetzte Dentalmaterial sowie die Größe der Kavität abgestimmt sein sollte. Für die Anwendung bei der Aushärtung entsprechender Dentalmaterialien geeignete Strahlungsvorrichtungen, welche vom Fachmann auch als Lichtgeräte bezeichnet werden, sind aus dem Stand der Technik bekannt und von verschiedenen Herstellern kommerziell verfügbar, wobei für dentale Anwendungen insbesondere Lichtgeräte für die Emission von elektromagnetischer Strahlung mit einer Wellenlänge im blauen Bereich gebräuchlich sind.

Technisch wurden früher zumeist Halogenlampen mit passendem Lichtfilter, bspw. Blaulichtfilter, als Strahlungsquelle in entsprechenden Lichtgeräten eingesetzt. Im Zuge der fortschreitenden technischen Entwicklung werden heutzutage jedoch vorrangig LEDs mit geeigneten Emissionswellenlängenbereichen eingesetzt.

Typische kommerziell erhältliche Geräte unterscheiden sich hinsichtlich der jeweiligen konstruktiven Ausgestaltung und der Optik, unterliegen hinsichtlich der Bauweise jedoch zumeist jeweils dem Anwendungserfordernis, dass das entsprechende Licht im Mund ergonomiegerecht abgestrahlt werden muss. Mit anderen Worten muss die zur Aushärtung der polymerisierbaren Dentalmaterialien benötigte elektromagnetische Strahlung effizient und komfortabel im Mund des Patienten applizierbar sein, insbesondere auch im Bereich schwer zugänglicher Zähne und/oder Positionen der zu füllenden Kavitäten.

In der Praxis liegt der Belichtungsort in der Zahnheilkunde, nämlich selten frei zugänglich, sondern erfordert einen guten Zugang mit der Strahlungsvorrichtung zwischen den Zähnen in schlecht zugänglichen Bereichen und gegebenenfalls ungünstigen Winkeln. Um das für die Polymerisation notwendige Licht mit der erforderlichen Qualität, d. h. geeignete Wellenlänge und Intensität, bedarfsgerecht an den Applikationsort zu bringen, werden im Stand der Technik häufig gebogene Lichtleiter eingesetzt. Bei diesem Ansatz ist die Strahlungsquelle regelmäßig im hinteren Teil der zumeist länglichen Strahlungsvorrichtung positioniert, was hinsichtlich der Steuerung, Energieversorgung und insbesondere zur Wärmeableitung von der eingesetzten Strahlungsquelle vorteilhaft ist. Über einen geeigneten Strahlungspfad wird die von der Strahlungsquelle erzeugte elektromagnetische Strahlung dann in einen gekrümmten Lichtleiter eingebracht, welcher das obere Ende der länglichen Strahlungsvorrichtung bildet, welches zur Anwendung in den Mund des Patienten eingeführt wird.

Der Lichtleiter ist hierbei gekrümmt, um den Strahlungsgang der elektromagnetischen Strahlung so abzulenken, dass auch anspruchsvolle Winkel im Mundraum realisiert werden können. Auch wenn mit diesem Typ von Strahlungsvorrichtungen im Stand der Technik regulär akzeptable Ergebnisse erzielt werden können, wird die entsprechende Bauform im Bereich der Technik doch zuweilen auch als nachteilig empfunden. Beispielsweise führt der gekrümmte Lichtleiter am Ende der Strahlungsvorrichtung in vielen Fällen dazu, dass der in den Mundraum eingeführte Teil der Strahlungsvorrichtung relativ groß ist, d.h. eine große Bauhöhe aufweist, was die Handhabung erschweren kann und von dem Patienten als unangenehm empfunden werden kann. Zudem wird zumeist eine starke Biegung des Lichtleiters angestrebt, da ein kleiner Krümmungsradius bevorzugt ist. Dies kann jedoch zu einer unerwünschten Verringerung der Lichtintensität führen, wodurch die Energieeffizienz bzw. die maximal erreichbare Strahlungsleistung nachteilig beeinflusst werden.

Zur Umgehung der vorstehend beschriebenen Probleme wurde eine alternative Bauform vorgeschlagen, welche durch den Einsatz von miniaturisierten LED-Bauelementen als Strahlungsquelle möglich wurde. Hierbei werden die Halbleiter der LED-Bauelemente direkt unter einem seitlichen Lichtaustrittsfenster implementiert und liegen entsprechend unmittelbar in dem Teil der Strahlungsvorrichtung, welcher in den Mund des Patienten eingeführt und in die Nähe der Kavität gebracht wird. Hierbei können in vorteilhafter Weise größere Emissionswinkel von beispielsweise 90° (bezogen auf die Längsachse der Strahlungsvorrichtung) erreicht werden, wobei sich in vorteilhafter Weise zudem eine geringere Bauhöhe ergibt, welche im Wesentlichen von der Größe der eingesetzten Elektronik bestimmt wird. Auch wenn entsprechende Aufbauten in vielerlei Hinsicht als vorteilhaft angesehen werden, werden sie hinsichtlich bestimmter Aspekte auch als nachteilig erachtet. Technisch ist insbesondere die ausreichende Wärmeableitung beim Betrieb der Halbleiterbauelemente, insbesondere bei hohen Emissionsleistungen, in vielen Fällen technisch schwierig. Problematisch ist zudem auch die zumeist unerwünscht hohe Divergenz des abgestrahlten Lichtes, sodass die gewünschte hohe Lichtintensität zumeist nur in unmittelbarem Abstand vom Lichtaustrittsfenster erreicht wird und die gewünschte Homogenität der Strahlungsleistung nur schwer umgesetzt werden kann.

Die Kompensation der vorstehend beschriebenen Problematiken erfordert dabei zumeist technisch aufwendige Lösungen, beispielsweise ein ausgefeiltes Kühlsystem zur Wärmeableitung oder den Einsatz von Laserdioden, welche die notwendige Lichtintensität auch im größeren Abstand erzeugen können und somit auch für die Aushärtung von Dentalmaterialien in tiefen Kavitäten geeignet sind. Diese technischen Lösungen sind, wie auch der grundsätzliche Aufbau unter Verwendung von miniaturisierten LED-Bauelementen, jedoch regelmäßig mit hohen Anforderungen an die eingesetzten Bauteile und deren Komplexität verbunden. Hierdurch steigen die Kosten für die eingesetzten Komponenten ebenso wie der Fertigungsaufwand bei der Herstellung entsprechender Lichtgeräte, sodass diese an sich technisch vorteilhaften Lösungen in den meisten Fällen nicht in zeit- und kosteneffizienter Art und Weise herstellbar sind.

Zu den beschriebenen Aspekten kommt zudem hinzu, dass teilweise auch mit Blick auf die Haltbarkeit entsprechender Strahlungsvorrichtungen gewisse Vorbehalte bestehen. Aus hygienischen Gründen müssen insbesondere die Teile der eingesetzten Strahlungsvorrichtung, welche im Einsatz in den Mund des Patienten eingeführt werden, regelmäßig besonders gründlich gereinigt bzw. sterilisiert werden. In der Praxis ist es vor diesem Hintergrund entsprechend häufig wünschenswert, mit mehrteilig ausgebildeten Strahlungsvorrichtungen zu arbeiten, die über einen Auswechselmechanismus dazu eingerichtet sind, dass die in den Mundraum eingebrachten Teile schnell und problemlos ausgetauscht werden können, um nach der Behandlung eines ersten Patienten durch den Austausch der kontaminierten Bauteile ohne Zeitverzögerung eine frische Strahlungsvorrichtung erzeugen zu können, während das zuvor benutzte Bauteil den vorgesehenen Aufbereitungsverfahren unterzogen wird. Bei der vorstehend beschriebenen Ausgestaltung, bei der sich die Strahlungsquelle sowie große Teile der notwendigen Elektronik und gegebenenfalls ein komplexes Kühlsystem innerhalb des Abschnitts der Strahlungsvorrichtung befinden, welcher ausgewechselt und aufbereitet werden müsste, werden quasi die teuersten und anfälligsten Bauteile den Belastungen des Aufbereitungsprozesses unterworfen, was mit Blick auf deren Lebensdauer nicht wünschenswert ist.

Beispielhafte Offenbarungen zu den vorstehend beschriebenen Strahlungsvorrichtungen finden sich beispielsweise in der US 9072572 B2 oder der US 2022/0202547 A1. Die EP 3442464 B1 offenbart eine zahnärztliche Lichtpolymerisierungsvorrichtung, welche einen aktiv bewegbaren Lichtreflektor umfasst, mit dem zwischen einer Bestrahlung des Zahns und einer Kameraaufnahme des Zahns gewechselt werden kann. Die EP 3122229 B1 offenbart eine Intraorale Bildgebungs- und Beleuchtungsvorrichtung, bei der die essentielle Elektronik in den hinteren Teil der Vorrichtung verlegt wird. Die US 2022/273399 A1 offenbart eine Vorrichtung, welche die Funktionalität eines therapeutischen Lasers und eines Polymerisationslichts vereint.

Vielen der aus dem Stand der Technik bekannten Strahlungsvorrichtungen für den Einsatz im dentalen Bereich ist gemein, dass die erzeugte Lichtemission zumeist nicht besonders flexibel an den Bedarf der Behandlungssituation angepasst werden kann, insbesondere hinsichtlich der Größe des Belichtungsfeldes, die bei dem aus dem Stand der Technik bekannten Lösungen zumeist konstant gehalten wird bzw. zumindest nicht während des Einsatzes unkompliziert angepasst werden kann.

Nach Einschätzung des Erfinders haben die aus dem Stand der Technik bekannten Strahlungsvorrichtungen zudem den Nachteil, dass in diesen keine effiziente, insbesondere keine permanente verfügbare, Überwachung der Strahlungsleistung möglich ist, insbesondere nicht während des Einsatzes im Mund des Patienten, was für eine effiziente Qualitätssicherung nachteilig ist. Sofern im Stand der Technik überhaupt Mittel für die Überprüfung der erbrachten Lichtintensität bereitgestellt werden, sind diese meist als separate Vorrichtungen ausgeführt und erfordern vom Anwender einen separaten Prüfungsschritt, worunter die Anwenderakzeptanz für eine häufige Durchführung einer an sich wünschenswerten regelmäßigen Überprüfung und gegebenenfalls Korrektur der Bestrahlung leiden kann.

Die primäre Aufgabe der vorliegenden Erfindung war es, die Nachteile des Standes der Technik auszuräumen oder zumindest abzuschwächen.

Insbesondere war es die Aufgabe der vorliegenden Erfindung, eine vorteilhafte Strahlungsvorrichtung für den Einsatz zur Aktivierung von polymerisierbaren Dentalmaterialien anzugeben, welche eine zuverlässige und effiziente Aktivierung von polymerisierbaren Dentalmaterialien ermöglicht. Hierbei war es wünschenswert, dass die anzugebende Strahlungsvorrichtung eine möglichst geringe Bauhöhe aufweisen sollte und die zuverlässige Applikation von elektromagnetischer Strahlung auch an schwer zugänglichen Stellen im Mundraum ermöglichen sollte. Es war eine Aufgabe der vorliegenden Erfindung, dass die anzugebende Strahlungsvorrichtung eine hohe Flexibilität der erreichbaren Emissionswinkel bzw. der Größe des Belichtungsfeldes aufweisen sollte, wobei der Emissionswinkel und die Größe des Belichtungsfeldes wünschenswerterweise direkt im Behandlungseinsatz anpassbar sein sollten, um polymerisierbare Dentalmaterialien auch an besonders schwer zugängliche Stellen sowie in Kavitäten unterschiedlicher Größen optimal auszuhärten.

Es war eine Aufgabe der vorliegenden Erfindung, dass die anzugebende Strahlungsvorrichtung die elektromagnetische Strahlung mit einer hohen Lichtintensität bereitstellen können sollte, wobei es wünschenswert war, dass ein zur Realisierung dieser hohen Lichtintensitäten gegebenenfalls notwendiges Kühlsystem möglichst einfach ausführbar sein sollte.

Zudem war es eine Aufgabe der vorliegenden Erfindung, dass die anzugebende Strahlungsvorrichtung besonders robust ausführbar sein sollte, um eine hohe Lebensdauer zu erreichen, wobei wünschenswerterweise die wesentlichen elektronischen Komponenten so angeordnet sein sollten, dass diese nicht in etwaigen auswechselbaren Teilen der Strahlungsvorrichtung angeordnet werden müssen, welche nach dem Einsatz im Mund eines Patienten einer Aufbereitung mit anspruchsvollen thermischen und/oder mechanischen Belastungen unterzogen werden müssen.

Darüber hinaus war es eine Aufgabe der vorliegenden Erfindung, dass die anzugebende Strahlungsvorrichtung besonders zeit- und/oder kosteneffizient herstellbar sein sollte, wobei wünschenswerterweise der Bedarf an komplexer Mikroelektronik möglichst gering gehalten werden sollte.

Des Weiteren war es eine ergänzende Aufgabe der vorliegenden Erfindung, dass die anzugebende Strahlungsvorrichtung in besonders effizienter Weise eine Messung bzw. eine Überwachung der tatsächlich geleisteten Lichtintensität erlauben sollte, um insoweit die Möglichkeit zur Qualitätssicherung und die diesbezügliche Anwenderakzeptanz für Qualitätssicherungsmaßnahmen zu erhöhen, wobei wünschenswerterweise eine Prüfung der Lichtintensität auch unmittelbar vor bzw. während des Einsatzes zur Aktivierung von polymerisierbaren Dentalmaterialien möglich sein sollte, um durch die automatisierte bzw. integrierte Überwachung der emittierten Lichtintensität eine besonders hohe Prozesssicherheit zu gewährleisten, bevorzugt auch durch effiziente Messungen in unmittelbarer zeitlicher Nähe zur Anwendung.

Der Erfinder der vorliegenden Erfindung hat nunmehr gefunden, dass sich die vorstehend beschriebenen Aufgaben überraschenderweise lösen lassen, wenn in einer Strahlungsvorrichtung auf den Einsatz eines Spiegels zurückgegriffen wird, welcher in dem für das Einführen in den Mund des Patienten vorgesehenen Kopfabschnitt des Gehäuses integriert und so ausgerichtet wird, dass der Spiegel elektromagnetische Strahlung, welche von einer im Grundabschnitt angeordneten Strahlungsquelle emittiert wird, ablenkt, sodass die elektromagnetische Strahlung aus dem dafür vorgesehenen Strahlungsaustrittsfenster austreten kann, wie es in den Ansprüchen definiert ist.

Die entsprechende Ausgestaltung der Strahlungsvorrichtung ermöglicht es in vorteilhafter Weise, die Strahlungsquelle im unteren Grundabschnitt der Strahlungsvorrichtung anzuordnen, wodurch eine effiziente Kühlung möglich wird. In dem Teil der Strahlungsvorrichtung, welcher im Einsatz in den Mund des Patienten eingeführt wird, kann in vorteilhafter Weise nahezu vollständig auf aufwendige Elektronik verzichtet werden, wodurch ein besonders robuster Aufbau erhalten werden kann, wobei der entsprechende Kopfabschnitt auch bei häufigen Reinigungs- bzw. Sterilisationsmaßnahmen eine hohe Haltbarkeit zeigt, selbst bei hohen Temperaturen.

Dank des Einsatzes eines in dem Gehäuse integrierten Spiegels ist eine besonders niedrige Bauhöhe realisierbar, während gleichzeitig vorteilhaft hohe Emissionswinkel erreicht werden können, ohne dass es zu einem unerwünschten Verlust an Lichtintensität kommt. Der entsprechende Aufbau erfindungsgemäßer Strahlungsvorrichtung ist dabei auch besonders zeit- bzw. kosteneffizient herstellbar. Ein weiterer Vorteil der erfindungsgemäßen Strahlungsvorrichtung ist darin zu sehen, dass der Spiegel besonders leicht als verstellbarer Spiegel, insbesondere automatisch verstellbarer Spiegel, ausgeführt werden kann, wodurch mit einer vergleichsweise einfachen Steuerung eine hohe Flexibilität in der Ablenkung der elektromagnetischen Strahlung erreicht wird, sodass eine große Bandbreite an Emissionswinkeln einstellbar ist, insbesondere auch während der Anwendung im Mundraum. Hierdurch kann die Lichtemission effizient auch auf schwer zugängliche Stellen im Mundraum gerichtet werden.

Zusätzlich oder alternativ können beim Einsatz eines Spiegels auch besonders effizient optische Linsen eingesetzt werden, welche die Form des von der Strahlungsquelle erzeugten Strahls beeinflussen. Insbesondere wenn die erfindungsgemäße Strahlungsvorrichtung so ausgeführt wird, dass der Abstand zwischen der Strahlungsquelle und der Linse verstellbar ist, lässt sich in besonders effizienter Weise die Größe des Belichtungsfeldes bzw. mittelbar die Lichtintensität steuern.

Als besonders großen Vorteil erfindungsgemäßer Strahlungsvorrichtungen erachtet es der Erfinder, dass in diesen besonders einfach eine integrierte Überwachung der emittierten Lichtintensität integriert werden kann. Durch die Wahl einer geeigneten baulichen Ausgestaltung des Spiegels bzw. durch die Umsetzung einer Verstellbarkeit des Spiegels ist es selbst im Einsatz bei der Aushärtung polymerisierbarer Dentalmaterialien möglich, die elektromagnetische Strahlung bzw. einen Teil der elektromagnetischen Strahlung auf einen integrierten Photosensor zu lenken und dadurch eine effiziente Überprüfung der Lichtintensität zu erlauben.

Hinsichtlich des Einsatzes eines Spiegels knüpft die Erfindung dabei grob an eine Konzeptideen aus den späten 80er Jahren des letzten Jahrhunderts an, wie sie in der EP 0360959 A1 offenbart ist, die sich nach Erkenntnis des Erfinder aber nie am Markt durchgesetzt hat. In der EP 0360959 A1 wurde vorgeschlagen, einen relativ großen externer Spiegel mit einer Halterung auf den Lichtleiter einer Strahlungsvorrichtung aufzusetzen, der das aus dem Lichtleiter austretende Licht umlenken aber insbesondere auch dem Zahnarzt als Beobachtungsspiegel einen unmittelbaren Blick auf die bestrahlte Stelle geben sollte. Die resultierende Vorrichtung war nach Einschätzung des Erfinders aber insbesondere auch wegen der unvorteilhaften Bauhöhe und des zusätzlichen Handhabungsaufwandes eines separaten Bauteils, welches den Aufwand der Reinigung erhöhte, unvorteilhaft. Zudem war das Konzept eines separaten Aufsatzes nach Einschätzung des Erfinders auch nicht dazu geeignet, präzise Emissionswinkel einzustellen, sodass die tatsächliche Strahlungsleistung vom handwerklichen Geschick des Anwenders abhing. Dies könnte potentiell so weit gehen, dass ein unzureichend monierter Spiegel zu einer ungewollten Reflektion von Strahlung führen konnte, so dass es insbesondere bei energiereicher Strahlung auch zu einer ungewollten Strahlungsexposition mit Folgeschäden kommen konnte. Auch wenn in der Konzeptidee seinerzeit auch über eine Verstellbarkeit des Spiegels nachgedacht wurde, musste diese manuell erfolgen und war selbst unter Annahme einer präzisen Befestigung des Aufsatzes auf dem Strahlenleiter quasi nicht gezielt steuerbar, insbesondere nicht automatisch einstellbar. Insbesondere durch die Integration des Spiegels in das Gehäuse der Strahlungsvorrichtung in erfindungsgemäßen Strahlungsvorrichtungen werden die bestehenden Probleme der Konzeptidee in vorteilhafter Weise ausgeräumt, wobei in vorteilhafter Weise insbesondere in den Bereichen der Arbeitssicherheit, der Anwenderakzeptanz, des Patientenkomforts, der Aufreinigbarkeit, der Haltbarkeit, der Automatisierbarkeit und der Sensorintegration erhebliche Vorteile erreicht werden.

Die vorstehend genannten Aufgaben werden entsprechend durch den Gegenstand der Erfindung gelöst, wie er in den Ansprüchen definiert ist. Bevorzugte erfindungsgemäße Ausgestaltungen ergeben sich aus den Unteransprüchen und den nachfolgenden Ausführungen.

Solche Ausführungsformen, die nachfolgend als bevorzugt bezeichnet sind, werden in besonders bevorzugten Ausführungsformen mit Merkmalen anderer als bevorzugt bezeichneter Ausführungsformen kombiniert. Ganz besonders bevorzugt sind somit Kombinationen von zwei oder mehr der nachfolgend als besonders bevorzugt bezeichneten Ausführungsformen. Ebenfalls bevorzugt sind Ausführungsformen, in denen ein in irgendeinem Ausmaß als bevorzugt bezeichnetes Merkmal einer Ausführungsform mit einem oder mehreren weiteren Merkmalen anderer Ausführungsformen kombiniert wird, die in irgendeinem Ausmaß als bevorzugt bezeichnet werden. Merkmale bevorzugter Verfahren und Verwendungen ergeben sich aus den Merkmalen bevorzugter Strahlungsvorrichtungen.

Die Erfindung betrifft insbesondere eine Strahlungsvorrichtung für den Einsatz zur Aktivierung von polymerisierbaren Dentalmaterialien, umfassend:
i) ein Gehäuse, umfassend:
   i.a) einen Grundabschnitt, und
   i.b) einen mit dem Grundabschnitt verbundenen Kopfabschnitt, wobei das Gehäuse im Kopfabschnitt ein Strahlungsaustrittsfenster umfasst,
   ii) eine im Bereich des Grundabschnitts im Inneren des Gehäuses angeordnete Strahlungsquelle zur Emission von elektromagnetischer Strahlung, wobei die Strahlungsquelle das Intensitätsmaximum der Emission bei einer Wellenlänge λ im Bereich von 350 bis 600 nm aufweist, und
   iii) ein im Bereich des Kopfabschnitts im Inneren des Gehäuses angeordneter Spiegel,

wobei sich zwischen der Strahlungsquelle und dem Spiegel ein Strahlungspfad erstreckt, wobei die Strahlungsvorrichtung dazu eingerichtet ist, dass von der Strahlungsquelle emittierte elektromagnetische Strahlung durch den Strahlungspfad auf den Spiegel auftreffen kann, und
wobei der Spiegel so angeordnet ist, dass die aus dem Strahlungspfad auf den Spiegel auftreffende elektromagnetische Strahlung vom Spiegel so reflektiert wird, dass die reflektierte elektromagnetische Strahlung durch das Strahlungsaustrittsfenster aus dem Gehäuse austritt, wobei die Strahlungsvorrichtung zumindest einen Photosensor umfasst,
   - wobei der Spiegel zwischen dem Photosensor und der Strahlungsquelle angeordnet ist, wobei der Spiegel eine durchgehende Ausnehmung umfasst, wobei die Strahlungsvorrichtung so ausgelegt ist, dass ein Teil der aus dem Strahlungspfad auf den Spiegel auftreffende elektromagnetische Strahlung der Wellenlänge λ durch die durchgehende Ausnehmung hindurch auf den Photosensor geleitet wird,
      oder
   - wobei die Strahlungsvorrichtung so ausgelegt ist, dass die Position des Spiegels und/oder die Ausrichtung des Spiegels reversibel und zerstörungsfrei so geändert werden kann,
      x) dass die aus dem Strahlungspfad auf den Spiegel auftreffende elektromagnetische Strahlung so reflektiert wird, dass die reflektierte elektromagnetische Strahlung auf den Photosensor geleitet wird, oder
      y) dass die elektromagnetische Strahlung am Spiegel vorbei auf den Photosensor geleitet wird.

Die erfindungsgemäße Strahlungsvorrichtung ist für den Einsatz bei der Aktivierung von polymerisierbaren Dentalmaterialien geeignet. Dies bedeutet, dass sie dazu in der Lage ist, elektromagnetische Strahlung einer für die Aushärtung von polymerisierbaren Dentalmaterialien geeigneten Wellenlänge zu emittieren, insbesondere in einer Form, dass diese Strahlung auf eine Kavität in einem Zahn gerichtet werden kann, um dort eine Polymerisation eines polymerisierbaren Dentalmaterials zu bewirken, beispielsweise mittels radikalischer Polymerisation von (Meth)Acrylat-Verbindungen.

Unter Berücksichtigung der vorgesehenen Anwendung im Bereich der Zahnheilkunde wird die erfindungsgemäße Strahlungsvorrichtung konstruktiv bevorzugt so ausgelegt sein, dass sie komfortabel in einer Hand gehalten und in den Mund des Patienten eingeführt werden kann, ohne dass dies als zu unangenehm empfunden wird. Besonders relevant ist entsprechend eine erfindungsgemäße Strahlungsvorrichtung, wobei die Strahlungsvorrichtung eine tragbare Strahlungsvorrichtung ist. Beispielhaft ist dabei eine erfindungsgemäße Strahlungsvorrichtung, wobei die Strahlungsvorrichtung eine Länge im Bereich von 10 bis 40 cm, bevorzugt im Bereich von 15 bis 35 cm, besonders bevorzugt im Bereich von 20 bis 30 cm, aufweist. Beispielhaft ist ebenfalls eine erfindungsgemäße Strahlungsvorrichtung, wobei die Strahlungsvorrichtung ein Gewicht von weniger als 500 g, bevorzugt weniger als 400 g, besonders bevorzugt weniger als 300 g, ganz besonders bevorzugt im Bereich von 100 bis 250 g, aufweist.

Typisch ist eine erfindungsgemäße Strahlungsvorrichtung, wobei die Strahlungsvorrichtung eine längliche Strahlungsvorrichtung ist.

In vorteilhafter Weise erlaubt es der Aufbau erfindungsgemäßer Strahlungsvorrichtungen, Emissionswinkel von mehr als 90° ohne eine Krümmung zu realisieren, so dass durch eine gerade bzw. flache Ausgestaltung, beispielsweise nach Art eines Stabes, besonders kleine Bauhöhen realisiert werden können. Bevorzugt ist entsprechend auch eine erfindungsgemäße Strahlungsvorrichtung, wobei die Strahlungsvorrichtung einen länglichen Kopfabschnitt, bevorzugt einen geraden länglichen Kopfabschnitt, besonders bevorzugt einen im Wesentlichen zylinderförmigen Kopfabschnitt, aufweist. Das bedeutet in anderen Worten, dass eine erfindungsgemäße Strahlungsvorrichtung bevorzugt ist, wobei der Kopfabschnitt im Wesentlichen nicht gekrümmt ist.

Beispielhaft ist eine längliche erfindungsgemäße Strahlungsvorrichtung, wobei der Quotient aus der Länge der Strahlungsvorrichtung entlang der Längsrichtung geteilt durch die mittlere Breite der Strahlungsvorrichtung orthogonal zur Längsrichtung im Bereich von 15:1 bis 5:1, bevorzugt im Bereich von 13:1 bis 7:1, besonders bevorzugt im Bereich von 11:1 bis 9:1, liegt.

Zweckmäßigerweise kann die erfindungsgemäße Strahlungsvorrichtung durch weitere konstruktive Elemente für einen angenehmen und komfortablen Einsatz bei der Behandlung optimiert werden, beispielsweise durch das Vorsehen eines optionalen gepolsterten Handgriffs sowie einer beispielsweise eingefärbten teiltransparenten Kunststoffplatte, welche als Sichtschutz dient und die Augen des Behandlers während der Strahlungsaktivierung der Dentalmaterialien vor der emittierten Strahlung schützt. Beispielhaft ist entsprechend eine erfindungsgemäße Strahlungsvorrichtung, wobei die Strahlungsvorrichtung einen im Bereich des Grundabschnitts angeordneten Handgriff umfasst. Beispielhaft ist zudem eine erfindungsgemäße Strahlungsvorrichtung, wobei die Strahlungsvorrichtung einen flächigen transparenten Sichtschutz umfasst, wobei der transparente Sichtschutz für elektromagnetische Strahlung der Wellenlänge λ entlang der orthogonal zur Flächenausdehnung liegenden Dickenrichtung einen reduzierten Transmissionsgrad, bevorzugt einen Transmissionsgrad von 0,1 oder weniger, bevorzugt 0,05 oder weniger, besonders bevorzugt 0,01 oder weniger, aufweist, wobei der Sichtschutz bevorzugt im Bereich des Grundabschnitts am Gehäuse befestigt ist.

Es ist denkbar, die Energieversorgung für erfindungsgemäße Strahlungsvorrichtungen über ein elektrisches Kabel zu realisieren. Mit Blick auf einen möglichst handhabungsfreundlichen Einsatz der entsprechenden Strahlungsvorrichtung schlägt der Erfinder jedoch vor, die Strahlungsvorrichtung in einer kabellosen Variante auszuführen. Bevorzugt ist entsprechend eine erfindungsgemäße Strahlungsvorrichtung, wobei die Strahlungsvorrichtung eine Energiespeichereinheit zur Versorgung der Strahlungsvorrichtung mit elektrischer Energie umfasst, bevorzugt eine Batterie oder einen Akkumulator, bevorzugt eine Lithium-Ionen-Batterie, wobei die Energiespeichereinheit bevorzugt um Bereich des Grundabschnitts im Inneren des Gehäuses angeordnet ist.

Die erfindungsgemäße Strahlungsvorrichtung umfasst zunächst ein Gehäuse, in bzw. an dem die weiteren Bestandteile der Strahlungsvorrichtung angeordnet sind, wobei es insbesondere bei Komponenten, welche anfällig für mechanische Belastungen sind, bevorzugt ist, diese im Inneren des Gehäuses anzuordnen. Hierbei werden mit Gehäusen aus Metall nicht nur besonders vorteilhafte Schutzeigenschaften, sondern auch eine besonders vorteilhafte Reinigungsfähigkeit erzielt. Bevorzugt ist somit eine erfindungsgemäße Strahlungsvorrichtung, wobei das Gehäuse zumindest teilweise, bevorzugt vollständig, aus Kunststoff oder Metall, bevorzugt Metall, ausgebildet ist.

Gemäß der vorstehenden Definition umfasst das Gehäuse einen Grundabschnitt und einen damit verbundenen Kopfabschnitt. In Übereinstimmung mit dem fachmännischen Verständnis werden hierdurch zum Zwecke der Definition unterschiedliche Bereiche des Gehäuses voneinander unterschieden, ohne dass es sich zwangsläufig um separate Bauteile handeln muss. Wie auch bei den aus dem Stand der Technik bekannten dentalen Strahlungsvorrichtungen handelt es sich bei dem Kopfabschnitt um den Teil des Gehäuses, welcher dafür vorgesehen ist, bei der späteren Anwendung in den Mund des Patienten eingeführt zu werden, während der Grundabschnitt die übrigen Abschnitte des Gehäuses bezeichnet, insbesondere die Teile, die den Griffbereich der Strahlungsvorrichtung bilden, d. h. den Teil des Gehäuses, an dem die Strahlungsvorrichtung für den Einsatz gegriffen wird und in dem beispielweise eine Energiespeichereinheit oder die Steuerungselektronik vorgesehen werden.

Auch wenn es, wie vorstehend erläutert, möglich ist, dass auch für ein einstückig ausgebildetes Gehäuse ein Grundabschnitt und ein Kopfabschnitt definiert werden, trägt die vorstehend gewählte Bezeichnung der Abschnitte bereits dem Umstand Rechnung, dass es besonders bevorzugt ist, die Strahlungsvorrichtung und infolgedessen auch das Gehäuse mehrteilig auszuführen. Hierdurch ist es in vorteilhafter Weise möglich, den Kopfabschnitt, welcher im Einsatz in den Mund des Patienten eingeführt wird und entsprechend anschließend gereinigt bzw. sterilisiert werden muss, nach dem Einsatz der Strahlungsvorrichtung von dem Grundabschnitt zu trennen. Ausgehend vom Grundabschnitt, welcher die auch ohne Reinigung wiederverwertbaren Teile, insbesondere einen Großteil der notwendigen Elektronik, umfasst, kann dann durch den Austausch des Kopfabschnittes schnell und effizient die Betriebsbereitschaft der erfindungsgemäßen Strahlungsvorrichtung wiederhergestellt werden. Bevorzugt ist demgemäß eine erfindungsgemäße Strahlungsvorrichtung, wobei die Strahlungsvorrichtung bzw. das Gehäuse der Strahlungsvorrichtung mehrteilig, bevorzugt zweiteilig, ausgeführt ist, wobei ein erster Teil der Strahlungsvorrichtung den Grundabschnitt umfasst, wobei ein zweiter Teil der Strahlungsvorrichtung den Kopfabschnitt umfasst, wobei der erste Teil und der zweite Teil reversibel und zerstörungsfrei lösbar miteinander verbunden sind. Bevorzugt ist eine erfindungsgemäße Strahlungsvorrichtung, wobei der erste Teil und der zweite Teil über eine Steckverbindung oder eine Schraubverbindung, bevorzugt eine Steckverbindung, reversibel und zerstörungsfrei lösbar miteinander verbunden sind.

Wie vorstehend erläutert, ist der Kopfabschnitt dafür vorgesehen, in den Mund des Patienten eingeführt zu werden, um dort die Aktivierung eines polymerisierbaren Dentalmaterials, beispielsweise in einer Kavität, zu bewirken. In Übereinstimmung mit den Anforderungen dieses Anwendungseinsatzes umfasst der Kopfabschnitt hierfür ein Strahlungsaustrittsfenster, d. h. eine Öffnung oder einen transparenten Bereich, durch die elektromagnetische Strahlung aus dem Inneren der Strahlungsvorrichtung austreten kann. Dieses Strahlungsaustrittsfenster ist angesichts der angestrebten starken Ablenkung der elektromagnetischen Strahlung in der Praxis zumeist seitlich im Gehäuse angeordnet, beispielsweise in der seitlichen Mantelfläche eines zylinderförmigen Kopfabschnitts, und verläuft beispielsweise im Wesentlichen parallel zu der Längsachse der Strahlungsvorrichtung. Bevorzugt ist vor diesem Hintergrund auch eine erfindungsgemäße Strahlungsvorrichtung, wobei das Strahlungsaustrittsfenster an dem vom Grundabschnitt abgewandten Ende des Kopfabschnitts, insbesondere bezogen auf die Längsrichtung der Strahlungsvorrichtung seitlich, angeordnet ist.

Auch wenn es zumindest theoretisch denkbar wäre, das notwendige Strahlungsaustrittsfenster durch eine durchgehende Ausnehmung im Gehäuse zu realisieren, ist es für im Wesentlichen alle Ausführungsformen bevorzugt, das Strahlungsaustrittsfenster mit einer Schutzabdeckung zu versehen und entsprechend als transparenten Bereich in der Gehäusewandung auszuführen. Hierdurch ist es in vorteilhafter Weise möglich, einer Verschmutzung der im Inneren des Gehäuses angeordneten Optik, insbesondere des Spiegels vorzubeugen. Entsprechend wird insbesondere die Reinigung der Strahlungsvorrichtung deutlich erleichtert und die Langlebigkeit der Komponenten erhöht. Vor dem Hintergrund einer angestrebten hohen Energieeffizienz ist es dabei zweckmäßig, die eingesetzte Schutzscheibe im relevanten Strahlungsbereich hinreichend transparent auszubilden, sodass diese einen möglichst geringen Einfluss auf die emittierte Lichtintensität ausübt. Bevorzugt ist somit eine erfindungsgemäße Strahlungsvorrichtung, wobei das Strahlungsaustrittsfenster zumindest teilweise, bevorzugt vollständig, mit einer transparenten Schutzscheibe verschlossen ist, bevorzugt aus Glas oder Kunststoff. Bevorzugt ist dabei eine erfindungsgemäße Strahlungsvorrichtung, wobei die transparente Schutzscheibe für elektromagnetische Strahlung der Wellenlänge λ entlang der Verbindungsrichtung zwischen dem Strahlungsaustrittsfenster und dem Spiegel einen Transmissionsgrad von 0,95 oder mehr, bevorzugt 0,98 oder mehr, besonders bevorzugt 0,99 oder mehr, aufweist.

In der erfindungsgemäßen Strahlungsvorrichtung wird die elektromagnetische Strahlung, welche im späteren Einsatz zur Aktivierung der polymerisierbaren Dentalmaterialien eingesetzt werden soll, durch eine Strahlungsquelle bereitgestellt.

Die vorstehende Definition der Strahlungsquellen über das Intensitätsmaximum der Emission ist dabei in Übereinstimmung mit dem fachmännischen Verständnis zweckmäßig, da viele Strahlungsquellen, beispielsweise übliche Leuchten, in ihrem Emissionsspektrum Anteile in verschiedenen Wellenlängenbereichen aufweisen können, die jedoch häufig nicht für eine effiziente Aktivierung entsprechender polymerisierbarer Dentalmaterialien geeignet wären. Vielmehr ist es zur Aktivierung eines strahlungshärtenden Dentalmaterials, welches beispielsweise durch die Wahl der Photoinitiatoren auf die Aktivierung mit einer Wellenlänge λ_{DM} ausgelegt ist, zweckmäßig, die Strahlungsquelle so zu wählen, dass das Intensitätsmaximum der Emission bei einer Wellenlänge λ liegt, die hinreichend nah an λ_{DM} liegt, so dass die Intensität bei λ_{DM} noch hinreichend hoch ist, wobei es in Übereinstimmung mit dem fachmännischen Verständnis besonders energieeffizient und zweckmäßig ist möglichst λ = λ_{DM} einzustellen. Der Fachmann versteht im Lichte der vorstehenden Definition entsprechend zwanglos, dass es sich beispielsweise für den Fall, dass λ bei 450 nm liegt, bei der Strahlungsquellen um einen blauen Strahler handelt, dessen Emissionsspektrum elektromagnetische Strahlung einer Wellenlänge λ von 450 nm nicht nur als Nebenbestandteil umfasst, sondern vielmehr die maximale Intensität der Emission bei dieser Wellenlänge aufweist. Bevorzugt ist nach Einschätzung des Erfinders für den angestrebten Einsatz im dentalen Bereich eine erfindungsgemäße Strahlungsvorrichtung, wobei die Strahlungsquelle das Intensitätsmaximum der Emission bei einer Wellenlänge λ im Bereich von 380 bis 550 nm, bevorzugt im Bereich von 400 bis 500 nm, besonders bevorzugt im Bereich von 440 bis 490 nm, ganz besonders bevorzugt im Bereich von 450 bis 480 nm, aufweist.

Für den Einsatz in erfindungsgemäßen Strahlungsvorrichtungen eignen sich nach Einschätzung des Erfinders insbesondere LEDs, da diese insbesondere hinsichtlich der Charakteristik des Emissionsspektrums besonders präzise eingestellt werden können, einen engen Wellenlängenbereich aufweisen, sehr energieeffizient betrieben werden können und zudem zumeist nur wenig Bauraum benötigen. Es kann dabei als Vorteil erfindungsgemäßer Strahlungsvorrichtungen gesehen werden, dass diese bereits mit typischen LED ausgezeichnete Strahlungseigenschaften realisieren können, wobei insbesondere durch den Einsatz von Linsen, wie er nachfolgend offenbart wird, auch eine effiziente Bündelung und eine niedrige Divergenz sichergestellt werden kann. Insbesondere für Hochleistungsanwendungen, beispielweise zur Aktivierung von polymerisierbaren Dentalmaterialien in besonders tiefen Kavitäten kann jedoch auch der Einsatz von Laser-LED mit Vorteilen verbunden sein. Für im Wesentlichen alle Ausführungsformen bevorzugt ist entsprechend eine erfindungsgemäße Strahlungsvorrichtung, wobei die Strahlungsquelle eine LED-Strahlungsquelle, bevorzugt eine leistungsfähige LED-Strahlungsquelle, insbesondere eine Laser-LED, ist.

Ein besonders wesentlicher Bestandteil erfindungsgemäßer Strahlungsvorrichtungen ist der Spiegel, welcher erfindungsgemäß im Inneren des Gehäuses angeordnet ist, nämlich im Kopfabschnitt, und dazu dient, die von der Strahlungsquelle erzeugte elektromagnetische Strahlung so zu reflektieren, dass sie aus dem Strahlungsaustrittsfenster aus dem Gehäuse austreten kann. Geeignete Spiegel sind von verschiedenen Herstellern kommerziell erhältlich und können prinzipiell in verschiedenen Ausgestaltungen realisiert werden, wobei es für die Praxis vor allem darauf ankommt, dass der Spiegel eine reflektierende Fläche bereitstellt, die zur Reflexion der einfallenden Strahlung geeignet ist, ohne dass die reflektierte elektromagnetische Strahlung über ein vernachlässigbares Maß hinaus ihre Parallelität verliert, d. h. dass es nur zu einem unwesentlichen Teil zu einer ungeordneten Streuung kommt. Bei entsprechenden Spiegeln kann es sich beispielsweise um Substrate handeln, welche mit einer reflektierenden Beschichtung, beispielsweise aus Metall, beschichtet sind.

Der Fachmann versteht, dass es zwar denkbar aber nicht bevorzugt wäre, eine Kette von Spiegeln hintereinander zu positionieren, um die gewünschte Ablenkung zu realisieren, da dadurch der konstruktive Aufbau und die Fehleranfälligkeit sowie die Gefahr für Intensitätsverluste zunehmen. Bevorzugt ist entsprechend eine erfindungsgemäße Strahlungsvorrichtung, wobei die Strahlungsvorrichtung im Kopfabschnitt genau einen oder zwei, bevorzugt genau einen, Spiegel umfasst und wobei der eine oder die zwei Spiegel so angeordnet sind, dass die aus dem Strahlungspfad austretende elektromagnetische Strahlung lediglich durch einfache oder zweifache, bevorzugt lediglich einfache, Reflexion durch das Strahlungsaustrittsfenster aus dem Gehäuse austritt.

Bei den in erfindungsgemäßen Strahlungsvorrichtungen eingesetzten Spiegeln handelt es sich um Spiegel im engeren Sinne, wobei die Reflexion der elektromagnetischen Strahlung an der reflektierenden Schicht erfolgt, die der Spiegel aufweist. Konstruktiv sind nach Einschätzung des Erfinders hierbei insbesondere Planspiegel oder Parabolspiegel bevorzugt. Im Rahmen der vorliegenden Erfindung werden sonstige Bauteile, welche zu einer Ablenkung der elektromagnetischen Strahlung führen könnten, insbesondere infolge unterschiedlicher Brechungsindizes, wie beispielsweise Prismen, nicht als Spiegel verstanden. Insbesondere fallen Lichtleiter, insbesondere gekrümmte Lichtleiter, wie sie im Stand der Technik verwendet werden, in Übereinstimmung mit dem fachmännischen Verständnis nicht unter die Definition eines Spiegels, wie er für die erfindungsgemäßen Strahlungsvorrichtungen einzusetzen ist. Der Fachmann versteht dabei zwanglos, dass der erfindungsgemäße Einsatz eines Spiegels im Kopfabschnitt eine vorteilhafte Alternative zum Einsatz eines Lichtleiters ist. Bevorzugt ist eine erfindungsgemäße Strahlungsvorrichtung, wobei der Spiegel ein Planspiegel oder ein Parabolspiegel, bevorzugt ein Planspiegel, ist. In Übereinstimmung mit dem fachmännischen Verständnis handelt es sich um eine erfindungsgemäße Strahlungsvorrichtung, wobei der Spiegel kein Lichtwellenleiter ist und keinen Lichtwellenleiter umfasst.

Der Aufbau erfindungsgemäßer Strahlungsvorrichtungen erlaubt es dank des im Inneren des Gehäuses verbauten Spiegels in besonders vorteilhafter Weise große Emissionswinkel zu realisieren, ohne dass die Bauhöhe unnötig stark erhöht werden muss. Bevorzugt ist zur Ausnutzung dieses Vorteils auch eine erfindungsgemäße Strahlungsvorrichtung, wobei der Spiegel so angeordnet ist, dass aus dem Strahlungspfad auf den Spiegel auftreffende elektromagnetische Strahlung der Wellenlänge λ um einen Winkel im Bereich von 60° bis 120°, bevorzugt im Bereich von 70° bis 110°, bevorzugt im Bereich von 80° bis 100°, abgelenkt wird.

Die relative Anordnung der Strahlungsquelle und des Spiegels wird in der vorstehenden Definition insbesondere über die Anordnung der jeweiligen Komponenten im Grundabschnitt bzw. im Kopfabschnitt definiert. Der Fachmann versteht insoweit, dass ein funktionelles Erfordernis darin besteht, dass die von der Strahlungsquelle erzeugte elektromagnetische Strahlung auch auf den Spiegel auftreffen kann und infolge der Reflexion durch das Strahlungsaustrittsfenster aus dem Gehäuse austreten kann. Diese Notwendigkeit wird in der vorstehenden Definition dadurch ausgedrückt, dass zwischen der Strahlungsquelle und dem Spiegel ein Strahlungspfad verläuft, durch den von der Strahlungsquelle emittierte elektromagnetische Strahlung zum Spiegel gelangen kann. Im aktivierten Zustand der Strahlungsvorrichtung, d. h. mit angeschalteter Strahlungsquelle, wird der Strahlengang der elektromagnetischen Strahlung durch den Strahlungspfad verlaufen und aus diesem auf den Spiegel auftreffen, um dort so reflektiert zu werden, dass der reflektierte Teil des Strahlenganges durch das Strahlungsaustrittsfenster verläuft. Da die Strahlungsquelle im Grundabschnitt und der Spiegel im Kopfabschnitt angeordnet sind, ist für den Fachmann klar, dass der Strahlungspfad sich entsprechend durch beide Abschnitte erstreckt. Mit anderen Worten handelt es sich um eine erfindungsgemäße Strahlungsvorrichtung, wobei der Strahlungspfad teilweise im Grundabschnitt und teilweise im Kopfabschnitt verläuft.

Auch wenn es denkbar ist, dass der Strahlungspfad beispielsweise durch eine Ausnehmung im Gehäuse von außen zugänglich ist, spricht nach Einschätzung des Erfinders wenig für eine entsprechende Ausgestaltung. Zumindest theoretisch ließe sich zudem auch ein Strahlungspfad realisieren, welcher beispielsweise durch den Einsatz von zusätzlichen Spiegeln nicht exakt gerade verläuft. Da nach Einschätzung der Erfinder jedoch keine Gründe für eine entsprechende Führung des Strahlengangs sprechen und vielmehr die Komplexität und die Anfälligkeit des Aufbaus unnötig erhöht würde, ist es für im Wesentlichen alle Ausführungsformen bevorzugt, wenn der Strahlungspfad zwischen der Strahlungsquelle und dem Spiegel gerade verläuft und entsprechend eine direkte und ungehinderte Bestrahlung des Spiegels ermöglicht. Bevorzugt ist entsprechend eine erfindungsgemäße Strahlungsvorrichtung, wobei der Strahlungspfad im Inneren des Gehäuses verläuft. Bevorzugt ist zusätzlich oder alternativ eine erfindungsgemäße Strahlungsvorrichtung, wobei der Strahlungspfad zwischen der Strahlungsquelle und dem Spiegel im Wesentlichen gerade verläuft.

Auch wenn es zumindest theoretisch denkbar wäre, den Strahlungspfad bis zum Spiegel weitgehend durch ein festes transparentes Medium, beispielsweise durch einen Glas- oder Kunststoffstrang zu realisieren, wäre dies nach Einschätzung des Erfinders mit Blick auf die am Ende resultieren Lichtintensität, sowie insbesondere bezüglich des resultierenden Gewichtes der Strahlungsvorrichtung, nicht nur unnötig, sondern auch weniger bevorzugt. Vielmehr schlagen die Erfinder vor, dass eine konstruktiv besonders bevorzugte Ausgestaltung realisierbar ist, wenn der Strahlungspfad als durchgehende Ausnehmung im Inneren des Gehäuses gebildet wird, welche beispielsweise mit Luft gefüllt ist und entsprechend den Durchtritt der elektromagnetischen Strahlung erlaubt. Mit anderen Worten handelt es sich um eine erfindungsgemäße Strahlungsvorrichtung, wobei der Strahlungspfad durch ein transparentes Medium gebildet wird, beispielsweise Luft, wobei der Strahlungspfad bevorzugt zumindest teilweise durch eine durchgehende Ausnehmung im Inneren des Gehäuses gebildet wird.

Die Ausführung des Strahlungspfades als durchgehende Ausnehmung zwischen der Strahlungsquelle und dem Spiegel lässt sich insbesondere bei einstückigen Strahlungsvorrichtungen besonders effizient umsetzen. Bei einer bevorzugten mehrteiligen Ausführung verläuft jedoch auch der Strahlungspfad in unterschiedlichen Teilen der Strahlungsvorrichtung und wäre beim Auswechseln des Kopfabschnitts entsprechend zugänglich. Insoweit gelten die vorstehenden Ausführungen zu der Zweckmäßigkeit eines Verschlusses des Strahlungsaustrittsfensters analog, da auch eine Verschmutzung des Strahlungspfades bei Trennung der Teile der mehrteiligen Strahlungsvorrichtung vermieden und die darin jeweils angeordneten Komponenten, d. h. die Strahlungsvorrichtung und der Spiegel, geschützt werden sollen. Bevorzugt ist entsprechend eine erfindungsgemäße Strahlungsvorrichtung, wobei der Strahlungspfad im Falle einer mehrteiligen Strahlungsvorrichtung eine oder mehrere transparente Verschlussscheiben umfasst, bevorzugt aus Glas oder Kunststoff, wobei die Verschlussscheiben bevorzugt im Verbindungsbereich zwischen dem ersten Teil und dem zweiten Teil angeordnet sind. Bevorzugt ist dabei wiederum eine erfindungsgemäße Strahlungsvorrichtung, wobei die transparente Verschlussscheiben für elektromagnetische Strahlung der Wellenlänge λ entlang der Richtung des Strahlungspfades einen Transmissionsgrad von 0,95 oder mehr, bevorzugt 0,98 oder mehr, besonders bevorzugt 0,99 oder mehr, aufweist.

Eine Ausgestaltung der erfindungsgemäßen Strahlungsvorrichtung mit einem fixen Spiegel ist nicht nur in der Fertigung besonders einfach herzustellen, sondern erweist sich im späteren Einsatz auch als besonders robust und fehlerunanfällig. Trotz dieses Umstands erachtet es der Erfinder für im Wesentlichen alle Ausführungsformen als besonders vorteilhaft, wenn der Spiegel als verstellbarer Spiegel, bevorzugt automatisch verstellbarer Spiegel, ausgeführt wird, da hierdurch eine besonders effiziente Manipulation des emittierten Strahlenganges möglich wird, insbesondere auch während des Einsatzes im Mund des Patienten. In vorteilhafter Weise kann hierbei durch die Spiegelstellung der Emissionswinkel, d.h. insbesondere die Ablenkung des Strahlengangs relativ zu der ursprünglichen Emissionsrichtung bzw. zur Längsachse der Strahlungsvorrichtung, beeinflusst werden. Wenn beispielsweise nach Ausrichtung des Lichtaustrittsfensters an die zu bestrahlende Kavität festgestellt wird, dass beispielsweise wegen einer schwierigen Zugänglichkeit und/oder einer ungünstigen Ausrichtung der Kavität eine optimale Bestrahlung mit dem eingestellten Emissionswinkel nicht erreicht werden kann, kann dieser besonders leicht angepasst werden, wobei es insbesondere auch möglich ist, diese zumindest teilweise entgegen der ursprünglich von der Strahlungsquelle erzeugten Strahlungsrichtung auszugeben, sodass insbesondere Hinterschnitte oder distale Kavitäten der Frontzähne besonders effizient bestrahlt werden können. Für im Wesentlichen alle Ausführungsformen bevorzugt ist eine erfindungsgemäße Strahlungsvorrichtung, wobei der Spiegel reversibel und zerstörungsfrei beweglich, bevorzugt reversibel und zerstörungsfrei rotierbar oder verschiebbar, besonders bevorzugt rotierbar, im Inneren des Gehäuses angeordnet ist. Bevorzugt ist dabei eine erfindungsgemäße Strahlungsvorrichtung, wobei der Spiegel um 20° oder mehr, bevorzugt 40° oder mehr rotierbar ist, und/oder wobei die Neigung des Spiegels relativ zum Strahlungspfad bevorzugt um 20° oder mehr, bevorzugt 40° oder mehr verändert werden kann. Bevorzugt ist mit anderen Worten eine erfindungsgemäße Strahlungsvorrichtung, wobei der Spiegel derart beweglich ist, dass aus dem Strahlungspfad auf den Spiegel auftreffende elektromagnetische Strahlung der Wellenlänge λ in Abhängigkeit von der Position des Spiegels in einem Winkelbereich von 80° bis 100°, bevorzugt im Winkelbereich von 70° bis 110°, besonders bevorzugt im Winkelbereich von 60° bis 120°, abgelenkt wird.

Eine besonders gute Strahlungsleistung mit vorteilhaften Strahlungscharakteristiken wird nach Erkenntnis der Erfinder dann erreicht, wenn im Strahlengang, d. h. konstruktiv im Strahlungspfad, eine Linse angeordnet wird, die vom Fachmann hinsichtlich ihrer Dimensionierung so gewählt werden kann, dass sie die von der Strahlungsquelle emittierte Strahlung in gewünschter Weise beeinflusst, beispielsweise durch eine Fokussierung in Richtung des Spiegels bzw. durch eine Orientierung bzw. Ausrichtung. Bevorzugt ist demgemäß eine erfindungsgemäße Strahlungsvorrichtung, wobei die Strahlungsvorrichtung zumindest eine im Strahlungspfad angeordnete Linse umfasst, die dazu eingerichtet ist, die von der Strahlungsquelle emittierte elektromagnetische Strahlung vor dem Auftritt auf den Spiegel zu beeinflussen, bevorzugt zu fokussieren. Bevorzugt ist dabei eine erfindungsgemäße Strahlungsvorrichtung, wobei die Linse im Bereich des Grundabschnitts im Inneren des Gehäuses angeordnet ist.

Eine bevorzugte Ausgestaltung, die in besonders bevorzugten Ausgestaltungen insbesondere auch mit einem verstellbaren Spiegel und insbesondere bevorzugt auch mit einer entsprechenden elektronischen Steuerung kombiniert wird, wird erhalten, wenn die Linse im Inneren der Strahlungsvorrichtung so angeordnet wird, dass der Abstand zwischen der Linse und der Strahlungsquelle verändert werden kann, wobei es nach Einschätzung der Erfinder bevorzugt ist, wenn insoweit die Linse beweglich ausgeführt wird und die Strahlungsquelle im Wesentlichen stationär verbleibt. Hierdurch ist es in vorteilhafter Weise möglich, die Größe des Belichtungsfeldes effizient anzupassen, insbesondere auch während des Einsatzes im Mundraum. Die Größe des Belichtungsfeldes kann dabei flexibel an den Bedarf der Behandlungssituation angepasst werden, beispielsweise durch die optimale Anpassung der Größe des Belichtungsfeldes an die Größe der zu bestrahlenden Kavität. Bevorzugt ist somit eine erfindungsgemäße Strahlungsvorrichtung, wobei die Strahlungsvorrichtung dazu eingerichtet ist, dass der Abstand zwischen der Linse und der Strahlungsquelle reversibel und zerstörungsfrei veränderbar ist. Besonders bevorzugt ist insoweit eine erfindungsgemäße Strahlungsvorrichtung, wobei die Linse in der Strahlungsvorrichtung reversibel und zerstörungsfrei bewegbar, bevorzugt relativ zur Strahlungsquelle entlang des Strahlungspfades bewegbar, angeordnet ist.

Als weitere besonders vorteilhafte Ausgestaltung erachten es die Erfinder, dass die erfindungsgemäße Strahlungsvorrichtung zudem ein Photosensor umfasst, mit dem die Lichtintensität bzw. ein mit der Lichtintensität korrelierende Parameter, beispielsweise eine infolge von Bestrahlung registrierte Stromstärke oder Spannung, gemessen werden kann. Geeignete Photosensoren sind dem Fachmann dabei ausgehend von seinem allgemeinen Fachwissen bekannt und von verschiedenen Herstellern kommerziell erhältlich, wobei die Photosensoren zweckmäßigerweise auf die jeweils verwendete Wellenlänge der elektromagnetischen Strahlung abgestimmt werden können. Erfindungsgemäß ist eine erfindungsgemäße Strahlungsvorrichtung, wobei die Strahlungsvorrichtung zumindest einen, bevorzugt im Inneren des Gehäuses angeordneten, Photosensor umfasst, bevorzugt eine Photodiode. In diesem Fall handelt es sich um eine erfindungsgemäße Strahlungsvorrichtung, wobei der Photosensor dazu eingerichtet ist, für auftreffende elektromagnetische Strahlung eine Intensitätsinformation zu bestimmen, wobei die Intensitätsinformation die Intensität der Strahlung und/oder einen mit der Intensität korrelierenden Parameter umfasst, insbesondere die Strahlungsleistung.

Nach Einschätzung des Erfinders ist es besonders vorteilhaft, die Lichtintensität möglichst unmittelbar vor dem Austritt der elektromagnetischen Strahlung aus der Strahlungsvorrichtung zu bestimmen. Bevorzugt ist entsprechend eine erfindungsgemäße Strahlungsvorrichtung, wobei der Photosensor im Bereich des Kopfabschnitts im Inneren des Gehäuses angeordnet ist.

Der Fachmann versteht, dass in dem vorstehend beschriebenen Aufbau aus Strahlungsquelle, Spiegel und Strahlungsaustrittsfenster im Betrieb ein Strahlengang ausgebildet wird, der nicht durch einen eingeschobenen Photosensor unterbrochen werden sollte. Entsprechend hat der Erfinder konstruktive Ausgestaltungen und Maßnahmen identifiziert, mit denen eine Messung der Strahlungsintensität während der Lichtapplikation möglich ist, bzw. sich während des Einsatzes schnell und effizient, beispielsweise in wiederkehrenden Abständen, durchführen lässt, wobei diese Lösungen in synergistischer Weise auf die Vorteile des Einsatzes eines Spiegels zurückgreifen. In einer ersten Ausgestaltung kann der zwischen dem Photosensor und der Strahlungsquelle angeordnete Spiegel mit einer Ausnehmung versehen werden, durch die ein Teil der Strahlung durch den Spiegel hindurch auf den Photosensor gelangen kann, sodass ein Anteil der elektromagnetischen Strahlung, der in der Praxis durch die Wahl eines sehr kleinen Loches möglichst gering gewählt werden wird, kontinuierlich für eine Messung der Strahlungsintensität zur Verfügung steht. Erfindungsgemäß ist in dieser Variante eine erfindungsgemäße Strahlungsvorrichtung, wobei der Spiegel zwischen dem Photosensor und der Strahlungsquelle angeordnet ist. Erfindungsgemäß ist insoweit eine erfindungsgemäße Strahlungsvorrichtung, wobei der Spiegel eine durchgehende Ausnehmung umfasst, wobei die Strahlungsvorrichtung so ausgelegt ist, dass ein Teil der aus dem Strahlungspfad auf den Spiegel auftreffende elektromagnetische Strahlung der Wellenlänge λ durch die durchgehende Ausnehmung hindurch auf den Photosensor geleitet wird.

Die vorstehend beschriebene Ausführungsform mit einer Ausnehmung im Spiegel ist nach Einschätzung des Erfinders insbesondere bei Einsatz eines stationären, d. h. nicht verstellbaren Spiegels zweckmäßig, kann jedoch dazu führen, dass es durch das Loch im Spiegel im Belichtungsfeld der Strahlungsvorrichtung zu einer Störstelle kommt, in der die Intensität infolge der fehlenden Reflexion verringert ist. Insoweit schlägt der Erfinder als besonders vorteilhaft vor, dass die ohnehin bevorzugte verstellbare Ausführung des Spiegels genutzt werden kann, um bedarfsgerecht, beispielsweise jeweils unmittelbar nach der Aktivierung der Strahlungsquelle, eine Messung durchzuführen. Hierbei bestehen zwei grundlegende Konzepte darin, dass der Spiegel entweder so verstellt werden kann, dass er den Strahlungspfad zwischen der Strahlungsquelle und dem Photosensor vorübergehend nicht blockiert oder so verstellt wird, dass der Spiegel die elektromagnetische Strahlung zeitweise nicht durch das Strahlungsaustrittsfenster reflektiert, sondern die Strahlung vielmehr auf den Photosensor zurückspiegelt, in dem diese dann vermessen werden kann. Erfindungsgemäß ist in dieser Variante eine erfindungsgemäße Strahlungsvorrichtung, wobei die Strahlungsvorrichtung so ausgelegt ist, dass die Position des Spiegels und/oder die Ausrichtung des Spiegels, bevorzugt die Ausrichtung des Spiegels, insbesondere die Rotationsposition, reversibel und zerstörungsfrei so geändert werden kann,
x) dass die aus dem Strahlungspfad auf den Spiegel auftreffende elektromagnetische Strahlung so reflektiert wird, dass die reflektierte elektromagnetische Strahlung auf den Photosensor geleitet wird, oder
y) dass die elektromagnetische Strahlung am Spiegel vorbei auf den Photosensor geleitet wird.

Insbesondere in Ausgestaltung erfindungsgemäßer Strahlungsvorrichtungen, in denen die zentralen Bauteile weitgehend stationär bzw. rotationsfest ausgeführt sind, insbesondere unter Einsatz eines stationären Spiegels in Verbindung mit einer stationären Linse, kann die notwendige Steuerungselektronik in vorteilhafter Weise sehr einfach gehalten werden, wodurch wiederum besonders robuste Strahlungsvorrichtungen erhalten werden, die zudem besonders kosteneffizient produziert werden können. Nach Einschätzung des Erfinders ist es jedoch für die weit überwiegende Zahl erfindungsgemäßer Strahlungsvorrichtungen bevorzugt, in diesen eine elektronische Datenverarbeitungsvorrichtung vorzusehen, die als Steuerungsvorrichtung dazu eingerichtet ist, die erfindungsgemäße Strahlungsvorrichtung zu steuern, und insbesondere die vorstehend beschriebenen Funktionalitäten zu ermöglichen, welche sich durch reversibel und zerstörungsfrei bewegliche Komponenten ergeben. Entsprechende hierfür geeignete Recheneinheiten bzw. Prozessoren sind, ebenso wie die notwendige Steuerungssoftware, von verschiedenen Anbietern kommerziell erhältlich bzw. vom Fachmann ohne größeren Aufwand an die von ihm jeweils benötigten Erfordernisse anzupassen. Für im Wesentlichen alle Ausführungsformen bevorzugt ist eine erfindungsgemäße Strahlungsvorrichtung, wobei die Strahlungsvorrichtung eine elektronische Datenverarbeitungsvorrichtung zur Steuerung und/oder Regelung der Strahlungsvorrichtung umfasst, wobei die elektronische Datenverarbeitungsvorrichtung bevorzugt im Bereich des Grundabschnitts im Inneren des Gehäuses angeordnet ist.

Die bevorzugt eingesetzte elektronische Datenverarbeitungsvorrichtung ist wünschenswerterweise dazu eingerichtet, die vorstehend beschriebenen Funktionalitäten sowie die generelle Strahlungsleistung zu steuern, beispielsweise automatisch, in Folge eines Sensorwertes, insbesondere des Photosensors, oder in Abhängigkeit von einer Eingabe des Anwenders. Bevorzugt ist somit zunächst eine erfindungsgemäße Strahlungsvorrichtung, wobei die elektronische Datenverarbeitungsvorrichtung dazu eingerichtet ist, die Strahlungsleistung der Strahlungsquelle reversibel und zerstörungsfrei zu verändern, bevorzugt in Folge einer manuellen Eingabe und/oder in Folge einer vom Photosensor bestimmten Intensitätsinformation. Bevorzugt ist zusätzlich oder alternativ eine erfindungsgemäße Strahlungsvorrichtung, wobei die elektronische Datenverarbeitungsvorrichtung dazu eingerichtet ist, die Position des Spiegels, insbesondere die Rotationsposition, und/oder die Ausrichtung des Spiegels, reversibel und zerstörungsfrei zu verändern, bevorzugt in Folge einer manuellen Eingabe, um den Ablenkungswinkel der reflektierten Strahlung zu verändern, bevorzugt in einem Winkelbereich von 80° bis 100°, besonders bevorzugt im Winkelbereich von 70° bis 110°, ganz besonders bevorzugt im Winkelbereich von 60° bis 120°. Bevorzugt ist wiederum zusätzlich oder alternativ auch eine erfindungsgemäße Strahlungsvorrichtung, wobei die elektronische Datenverarbeitungsvorrichtung dazu eingerichtet ist, die Position der Linse relativ zu der Strahlungsquelle entlang des Strahlungspfades reversibel und zerstörungsfrei zu verändern, bevorzugt in Folge einer manuellen Eingabe und/oder in Folge einer vom Photosensor bestimmten Intensitätsinformation.

Bevorzugt ist des Weiteren zusätzlich oder alternativ zu den vorstehend offenbarten Ausführungsformen auch eine erfindungsgemäße Strahlungsvorrichtung, wobei die elektronische Datenverarbeitungsvorrichtung dazu eingerichtet ist, die Position des Spiegels, insbesondere die Rotationsposition, und/oder die Bewegung des Spiegels, bevorzugt die Rotationsposition des Spiegels, reversibel und zerstörungsfrei so zu verändern,
x) dass die aus dem Strahlungspfad auf den Spiegel auftreffende elektromagnetische Strahlung so reflektiert wird, dass die reflektierte elektromagnetische Strahlung auf den Photosensor geleitet wird, oder
y) dass die elektromagnetische Strahlung am Spiegel vorbei aus dem Strahlungspfad auf den Photosensor geleitet wird.

Bevorzugt ist beim Einsatz eines Photosensors eine erfindungsgemäße Strahlungsvorrichtung, wobei die Strahlungsvorrichtung dazu eingerichtet ist, für die von der Strahlungsquelle emittierte elektromagnetische Strahlung im Betrieb kontinuierlich oder infolge einer vorbestimmten Auslösebedingung, bevorzugt zu einem vorgegebenen Zeitpunkt nach der Aktivierung oder infolge einer manuellen Eingabe, im Innenraum des Gehäuses mit dem Photosensor eine Intensitätsinformation zu bestimmen.

Bevorzugt ist beim Einsatz eines Photosensors die nachfolgend für das erfindungsgemäße Verfahren beschriebene Ausgestaltung, nämlich eine erfindungsgemäße Strahlungsvorrichtung, wobei die elektronische Datenverarbeitungsvorrichtung dazu eingerichtet ist, die Leistung der Strahlungsquelle und/oder der Abstand zwischen der Strahlungsquelle und der Linse in Abhängigkeit von der Intensitätsinformation zu steuern bzw. zu regeln.

Zur Steigerung der Betriebssicherheit kann die elektronische Datenverarbeitungsvorrichtung zudem darauf ausgelegt werden, Schutz- oder Korrekturmaßnahmen zu realisieren, wenn die vom Photosensor gemessene Intensität von der bestimmten Intensität abweicht, beispielsweise weil diese zu niedrig oder zu hoch ist. Bevorzugt ist hierfür eine erfindungsgemäße Strahlungsvorrichtung, wobei die elektronische Datenverarbeitungsvorrichtung dazu eingerichtet ist, eine von dem Photosensor bestimmte Intensitätsinformation mit einem vorgegebenen Referenzkriterium abzugleichen, wobei die elektronische Datenverarbeitungsvorrichtung dazu eingerichtet ist, Maßnahmen einzuleiten, wenn die Abweichung vom Referenzkriterium außerhalb eines vorgegebenen Toleranzbereiches liegt. Bevorzugt ist dabei eine erfindungsgemäße Strahlungsvorrichtung, wobei die Maßnahmen ausgewählt sind aus der Gruppe bestehend aus der Deaktivierung der Strahlungsvorrichtung und der Ausgabe eines Warnsignals, bevorzugt eines optischen und/oder akustischen Warnsignals.

Es kann als Vorteil erfindungsgemäßer Strahlungsvorrichtungen gesehen werden, dass diese besonders effizient auf hohe Strahlungsleistungen und Lichtintensitäten ausgelegt werden können, die auch für relativ lange Zeiten aufrechterhalten werden können. Entsprechend ist es auch bevorzugt, diese Eignung dazu zu nutzen, mit erfindungsgemäßen Strahlungsvorrichtungen hohe Intensitäten zu realisieren. Bevorzugt ist dabei insbesondere eine erfindungsgemäße Strahlungsvorrichtung, wobei die Strahlungsvorrichtung dazu eingerichtet ist, aus dem Strahlungsaustrittsfenster elektromagnetische Strahlung mit einer Intensität von 500 mW/cm² oder mehr, bevorzugt von 800 mW/cm² oder mehr, besonders bevorzugt von 1000 mW/cm² oder mehr, zu emittieren, bevorzugt für eine Zeit von 10 s oder mehr, besonders bevorzugt 15 s oder mehr, zu emittieren.

Insbesondere bei Einsatz hoher Intensitäten und/oder langer Belichtungsdauern ist es nach Einschätzung des Erfinders dabei bevorzugt, zudem Elemente zur Wärmeabfuhr von der Strahlungsquelle vorzusehen, wobei diese dank der Anordnung der Strahlungsquelle im Grundabschnitt in erfindungsgemäßen Strahlungsvorrichtungen besonders leicht und effizient einzubauen sind. Bevorzugt ist somit insbesondere für leistungsstarke Strahlungsvorrichtungen eine erfindungsgemäße Strahlungsvorrichtung, wobei die Strahlungsvorrichtung zumindest ein Element zur Wärmeabführung der Strahlungsquelle umfasst, bevorzugt ein passives Kühlelement.

Die Erfindung betrifft auch ein nicht-therapeutisches Verfahren zum Betrieb einer erfindungsgemäßen Strahlungsvorrichtung, umfassend die Verfahrensschritte:
a) Erzeugen von elektromagnetischer Strahlung mit der Strahlungsquelle, wobei die elektromagnetische Strahlung das Intensitätsmaximum bei einer Wellenlänge λ im Bereich von 350 bis 600 nm aufweist,
b) Leiten der elektromagnetischen Strahlung im Inneren des Gehäuses von der Strahlungsquelle zu dem Spiegel durch den Strahlungspfad, und
c) Ablenken der elektromagnetischen Strahlung mit dem Spiegel, so dass die reflektierte elektromagnetische Strahlung durch das Strahlungsaustrittsfenster aus dem Gehäuse austritt.

Bevorzugt sind Ausgestaltungen des erfindungsgemäßen Verfahrens, in denen die vorstehend offenbarten bevorzugten erfindungsgemäßen Strahlungsvorrichtungen eingesetzt und hinsichtlich der jeweiligen vorteilhaften Funktionalitäten dabei auch genutzt werden, beispielsweise zur Umsetzung hoher Strahlungsleistung, der Veränderbarkeit des Austrittswinkels aus dem Strahlungsaustrittsfenster oder die Anpassbarkeit des Belichtungsfeldes.

Bevorzugt ist somit zunächst ein erfindungsgemäßes Verfahren, wobei die aus dem Strahlungsaustrittsfenster austretende elektromagnetische Strahlung eine Intensität von 500 mW/cm² oder mehr, bevorzugt von 800 mW/cm² oder mehr, besonders bevorzugt von 1000 mW/cm² oder mehr, aufweist.

Bevorzugt ist zusätzlich oder alternativ auch ein erfindungsgemäßes Verfahren, wobei die Strahlungsvorrichtung für eine Zeit von 10 s oder mehr, bevorzugt 15 s oder mehr, besonders bevorzugt 20 s oder mehr, betrieben wird.

Bevorzugt ist zusätzlich oder alternativ auch ebenso ein erfindungsgemäßes Verfahren, wobei die Position des Spiegels verändert wird, um die Ablenkung der elektromagnetische Strahlung und den Austrittswinkel aus dem Strahlungsaustrittsfenster zu verändern, bevorzugt in Abhängigkeit von der Form und Position des zu bestrahlenden Substrates.

Bevorzugt ist des Weiteren zusätzlich oder alternativ auch ein erfindungsgemäßes Verfahren, wobei der Abstand zwischen der Strahlungsquelle und der Linse verändert wird, um für die aus dem Strahlungsaustrittsfenster austretende elektromagnetische Strahlung die Größe des Strahlungsquerschnitts und/oder die Leistungsdichte einzustellen, bevorzugt in Abhängigkeit von der Beschaffenheit des zu bestrahlenden Substrates und/oder den Materialeigenschaften des zu bestrahlenden Materials.

Bevorzugt ist wiederum zusätzlich oder alternativ zudem ein erfindungsgemäßes Verfahren, wobei das Verfahren zusätzlich einen der folgenden Verfahrensschritte umfasst, die in zeitlichen Abständen ausgeführt werden, bevorzugt in vorgegebenen zeitlichen Abständen:
d1) vorübergehendes Ändern der Position des Spiegels und/oder der Rotationsposition des Spiegels, so dass die elektromagnetische Strahlung so reflektiert wird, dass die reflektierte elektromagnetische Strahlung auf den Photosensor geleitet wird, oder
d2) vorübergehendes Ändern der Position des Spiegels und/oder der Rotationsposition des Spiegels, so dass die elektromagnetische Strahlung am Spiegel vorbei auf den Photosensor geleitet wird.

Bevorzugt ist zudem zusätzlich oder alternativ ein erfindungsgemäßes Verfahren, wobei die Leistung der Strahlungsquelle und/oder der Abstand zwischen der Strahlungsquelle und der Linse in Abhängigkeit von der Intensitätsinformation gesteuert bzw. geregelt werden.

Bevorzugt ist darüber hinaus zusätzlich oder alternativ ein erfindungsgemäßes Verfahren, wobei die von dem Photosensor bestimmte Intensitätsinformation mit einem vorgegebenen Referenzkriterium abgeglichen wird, wobei Maßnahmen eingeleitet werden, wenn die Abweichung vom Referenzkriterium außerhalb eines vorgegebenen Toleranzbereiches liegt.

Offenbart wird auch die Verwendung einer erfindungsgemäßen Strahlungsvorrichtung, zur Aktivierung von polymerisierbaren Dentalmaterialien bei der Herstellung und/oder Bearbeitung dentaler Restaurationen.

Bei der Entwicklung der Erfindung hat sich gezeigt, dass der vorstehend für erfindungsgemäße Strahlungsvorrichtungen offenbarte Einsatz eines integrierten Photosensors auch losgelöst vom erfindungsgemäß vorgesehenen Einsatz eines Spiegels vorteilhaft ist. Aufbauend auf dieser Erkenntnis wird nachfolgend zusätzlich oder alternativ zur erfindungsgemäßen Strahlungsvorrichtungen noch eine generell vorteilhafte Ausgestaltung einer Strahlungsvorrichtung offenbart. Zur klaren Unterscheidung von erfindungsgemäßen Strahlungsvorrichtungen wird diese Ausgestaltung nachfolgend als "dentales Lichtgerät" bezeichnet.

Offenbart wird zusätzlich oder alternativ zu erfindungsgemäßen Strahlungsvorrichtungen entsprechend ein dentales Lichtgerät für den Einsatz zur Aktivierung von polymerisierbaren Dentalmaterialien, umfassend:
i-2) ein Gehäuse, umfassend einen Innenraum mit einem Strahlungsauslass, bevorzugt mit einem Strahlungsaustrittsfenster,
ii-2) eine im Innenraum des Gehäuses angeordnete Strahlungsquelle zur Emission von elektromagnetischer Strahlung, wobei die Strahlungsquelle das Intensitätsmaximum der Emission bei einer Wellenlänge λ im Bereich von 350 bis 600 nm aufweist,
iii-2) einen im Innenraum des Gehäuses angeordneten Photosensor, bevorzugt eine Photodiode, und
iv-2) eine elektronische Datenverarbeitungsvorrichtung,

wobei das dentale Lichtgerät dazu eingerichtet ist, für die von der Strahlungsquelle emittierte elektromagnetische Strahlung im Betrieb kontinuierlich oder infolge einer vorbestimmten Auslösebedingung, bevorzugt zu einem vorgegebenen Zeitpunkt nach der Aktivierung oder infolge einer manuellen Eingabe, im Innenraum des Gehäuses mit dem Photosensor eine Intensitätsinformation zu bestimmen,
wobei die elektronische Datenverarbeitungsvorrichtung dazu eingerichtet ist, das dentale Lichtgerät in Abhängigkeit von der bestimmten Intensitätsinformation zu steuern und/oder zu regeln.

Besonders bevorzugt sind hierbei Ausgestaltungen des dentalen Lichtgerätes, in denen die Intensitätsinformation infolge einer vorbestimmten Auslösebedingung bestimmt wird. Bevorzugt ist zusätzlich oder alternativ ein dentales Lichtgerät, wobei das dentale Lichtgerät einen beweglichen Spiegel umfasst, so dass die elektromagnetische Strahlung infolge einer vorbestimmten Auslösebedingung auf den Photosensor abgelenkt werden kann und/oder wobei der Photosensor im Innenraum reversibel und zerstörungsfrei beweglich angeordnet ist, so dass der Photosensor infolge einer vorbestimmten Auslösebedingung in den Strahlengang der elektromagnetischen Strahlung gebracht werden kann.

Offenbart wird zudem eine Methode zum Betrieb eines dentalen Lichtgerätes, umfassend die Schritte:
aa) Erzeugen von elektromagnetischer Strahlung mit der Strahlungsquelle, wobei die elektromagnetische Strahlung das Intensitätsmaximum bei einer Wellenlänge λ im Bereich von 350 bis 600 nm aufweist, und
bb) Leiten der elektromagnetischen Strahlung im Innenraum des Gehäuses zum Strahlungsauslass,
wobei mit dem im Innenraum angeordneten Photosensor im Betrieb kontinuierlich oder infolge einer vorbestimmten Auslösebedingung, bevorzugt zu einem vorgegebenen Zeitpunkt nach der Aktivierung oder infolge einer manuellen Eingabe, mit dem im Innenraum angeordneten Photosensor eine Intensitätsinformation bestimmt wird, und
wobei das dentale Lichtgerät in Abhängigkeit von der bestimmten Intensitätsinformation gesteuert und/oder geregelt wird.

Merkmale bevorzugter dentaler Lichtgeräte und Methoden ergeben sich aus den Merkmalen erfindungsgemäßer Strahlungsvorrichtungen und erfindungsgemäßer Verfahren, wobei die Ausführungen zu den entsprechenden Vorteilen entsprechend zutreffen.

Nachfolgend werden die Erfindung und bevorzugte Ausführungsformen der Erfindung unter Bezugnahme auf die beiliegenden Figuren näher erläutert und beschrieben. Dabei zeigen:
- Fig. 1: eine schematische Querschnittsdarstellung einer erfindungsgemäßen Strahlungsvorrichtung in einer bevorzugten Ausführungsform;
- Fig. 2: eine schematische Visualisierung der Verstellbarkeit des Spiegels in erfindungsgemäßen Strahlungsvorrichtungen in bevorzugten Ausführungsformen; und
- Fig. 3: eine schematische Darstellung zur Realisierung der Bestrahlung eines Photosensors in erfindungsgemäßen Strahlungsvorrichtungen in bevorzugten Ausführungsform.

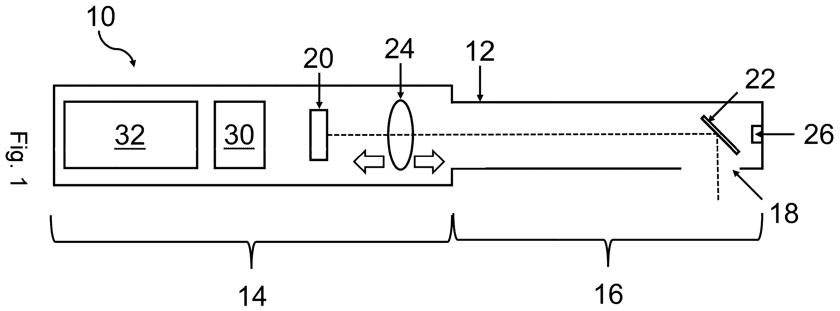

Fig. 1 zeigt eine schematische Querschnittsdarstellung durch eine erfindungsgemäße Strahlungsvorrichtung 10, welche für den Einsatz zur Aktivierung von polymerisierbaren Dentalmaterialien im Mundraum geeignet ist. Die Strahlungsvorrichtung 10 umfasst ein Gehäuse 12, in dem die Komponenten der Strahlungsvorrichtung 10 angeordnet sind. Das Gehäuse 12 umfasst dabei einen Grundabschnitt 14 und einen Kopfabschnitt 16, welche jeweils durch geschweifte Klammern eingetragen sind.

Die Strahlungsvorrichtung 10 der Fig. 1 ist zweistückig ausgeführt, wobei der den Grundabschnitt 14 bildende untere Teil und der für das Einführen in den Mund vorgesehene Kopfabschnitt 16 an der Grenzfläche reversibel und zerstörungsfrei lösbar durch eine Steckverbindung miteinander verbunden sind (nicht eingezeichnet).

Im Inneren des Gehäuses 12 ist eine Strahlungsquelle 20 angeordnet, deren Intensitätsmaximum der Emission im gezeigten Beispiel bei einer Wellenlänge λ von 450 nm liegt. Die Strahlungsvorrichtung 10 ist so ausgelegt, dass die von der Strahlungsquelle 20 erzeugte elektromagnetische Strahlung als gerader Strahl durch den Strahlungspfad auf den Spiegel 22 auftreffen kann, welcher im Kopfabschnitt 16 angeordnet ist. Der Strahlungspfad ist dabei im Wesentlichen als mit Luft gefüllte Ausnehmung im Inneren des Gehäuses 12 umgesetzt, die lediglich im Kontaktbereich der beiden Teile des Gehäuses jeweils mit einer im Wesentlichen transparenten Glasscheibe verschlossen ist (nicht gezeigt).

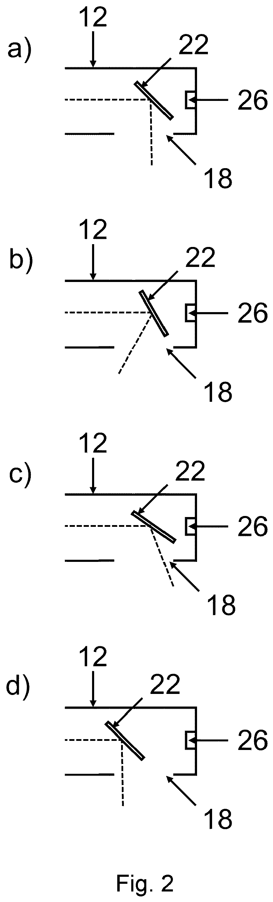

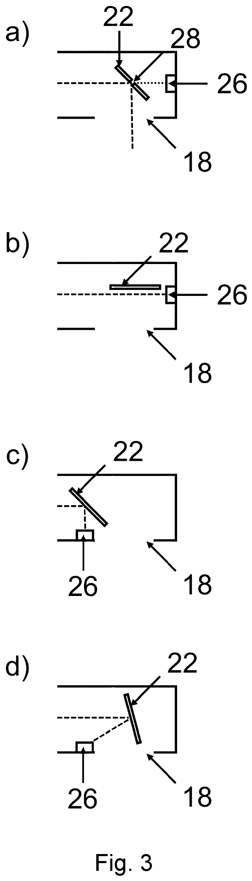

In Fig. 1 ist zu erkennen, wie die von der Strahlungsquelle 20 erzeugte elektromagnetische Strahlung, welche als gestrichelte Linie dargestellt ist, vom Spiegel 22 um etwa 90° reflektiert und abgelenkt wird, sodass die abgelenkte elektromagnetische Strahlung durch das Strahlungsaustrittsfenster 18 aus dem Gehäuse 12 austreten kann. In der bevorzugten Ausführungsform der Fig. 1 ist im Strahlungspfad, d. h. bei Aktivierung im Strahlengang der elektromagnetischen Strahlung, zudem eine Linse 24 angeordnet, welche reversibel und zerstörungsfrei entlang des Strahlenganges verschoben werden kann, um die von der Strahlungsquelle 20 emittierte elektromagnetische Strahlung so zu beeinflussen, dass die Größe des Belichtungsfeldes hierdurch eingestellt werden kann. Im Grundabschnitt 14 sind im Inneren des Gehäuses neben der Strahlungsquelle 20 und der Linse 24 zudem eine elektronische Datenverarbeitungsvorrichtung 30 und eine Energiespeichereinheit 32 angeordnet, wobei Letztere der Versorgung der erfindungsgemäßen Strahlungsvorrichtung 10 mit elektrischer Energie dient. Die elektronische Datenverarbeitungsvorrichtung 30 ist im gezeigten Beispiel dazu eingerichtet, nicht nur die Strahlungsleistung der Strahlungsquelle 20 zu steuern, sondern zudem auch die Position der Linse 24 zu verändern, insbesondere infolge der Eingabe eines Anwenders. Zudem ist die elektronische Datenverarbeitungsvorrichtung 30 dazu eingerichtet, den verstellbaren Spiegel 22 zu steuern, wodurch insbesondere die Veränderung des Emissionswinkels aus dem Strahlungsaustrittsfenster 18 sowie die Bestrahlung des Photosensors 26 realisiert wird, wie es in den Fig. 2 und 3 schematisch dargestellt ist.

Fig. 2 zeigt in vier vereinfachten Vergrößerungen des Kopfabschnitts 16 der erfindungsgemäßen Strahlungsvorrichtung 10 der Fig. 1 unterschiedliche Positionierungen des Spiegels 22, welche über die elektronische Datenverarbeitungsvorrichtung 30 eingestellt werden können. Die Fig. 2a) entspricht dabei der in Fig. 1 gezeigten Ausrichtung. In den Fig. 2b) und Fig. 2c) wird die Position des Spiegels jeweils durch Verkippen verändert, um den Emissionswinkel kleiner oder größer zu gestalten, wodurch in vorteilhafter Weise eine Anpassung an die jeweiligen Einsatzbedingungen erreicht werden kann. Im Gegensatz hierzu erfolgt in Fig. 2d) kein Verkippen, sondern eine translatorische Bewegung des Spiegels 22 entlang des Strahlungspfades. Hierdurch ist es in vorteilhafter Weise möglich, eine Feinjustierung der bestrahlten Position vorzunehmen, ohne dass die Strahlungsvorrichtung 10 als Ganzes bewegt werden muss.

Fig. 3 zeigt wiederum in vereinfachten Ausschnittsdarstellungen des Kopfabschnitts 16 verschiedene Möglichkeiten, um eine Messung der Strahlungsintensität der elektromagnetischen Strahlung mit dem Photosensor 26 zu realisieren. In Fig. 3a) ist der Spiegel 22 hierfür mit einer durchgehenden Ausnehmung 28 ausgerüstet, durch die ein Teil der elektromagnetischen Strahlung durch den Spiegel 22 hindurch auf den Photosensor 26 auftreffen kann, um dort vermessen zu werden. Fig. 3b) zeigt eine Ausgestaltung, in der die Verstellbarkeit des Spiegels, insbesondere die Verschwenkbarkeit des Spiegels ausgenutzt wird, um diesen bei Bedarf aus dem die Strahlungsquelle 20 und den Photosensor 26 verbindenden Strahlengang herauszubewegen, um beispielsweise infolge einer vom Anwender ausgelösten Qualitätssicherungsoperation die Strahlungsintensität zu bestimmen.

Im Unterschied hierzu zeigen die Fig. 3c) und 3d) Ausgestaltungen, in denen der Spiegel 22 dazu verwendet wird, um die elektromagnetische Strahlung auf den Photosensor 26 zu reflektieren. In Fig. 3c) gelingt dies durch eine translatorische Verschiebung des Spiegels 22 entlang des Strahlungspfades, um eine neben dem Strahlungsaustrittsfenster 18 angeordneten Photosensor 26 mit Strahlung zu beaufschlagen. In Fig. 3d) wird hingegen eine rotatorische Verstellung des Spiegels 22 ausgenutzt, um die reflektierte elektromagnetische Strahlung zeitweise nicht mehr durch das Strahlungsaustrittsfenster 18 aus dem Gehäuse 12 hinaus, sondern vielmehr auf den Photosensor 26 zu lenken.

### Bezugszeichenliste

- 10: Strahlungsvorrichtung
- 12: Gehäuse
- 14: Grundabschnitt
- 16: Kopfabschnitt
- 18: Strahlungsaustrittsfenster
- 20: Strahlungsquelle
- 22: Spiegel
- 24: Linse
- 26: Photosensor
- 28: Ausnehmung
- 30: Datenverarbeitungsvorrichtung
- 32: Energiespeichereinheit

## Patentansprüche

1. Strahlungsvorrichtung (10) für den Einsatz zur Aktivierung von polymerisierbaren Dentalmaterialien, umfassend:
i) ein Gehäuse (12), umfassend:
i.a) einen Grundabschnitt (14), und
i.b) einen mit dem Grundabschnitt (14) verbundenen Kopfabschnitt (16), wobei das Gehäuse (12) im Kopfabschnitt (16) ein Strahlungsaustrittsfenster (18) umfasst,
ii) eine im Bereich des Grundabschnitts (14) im Inneren des Gehäuses (12) angeordnete Strahlungsquelle (20) zur Emission von elektromagnetischer Strahlung, wobei die Strahlungsquelle (20) das Intensitätsmaximum der Emission bei einer Wellenlänge λ im Bereich von 350 bis 600 nm aufweist, und
iii) ein im Bereich des Kopfabschnitts (16) im Inneren des Gehäuses (12) angeordneter Spiegel (22),
wobei sich zwischen der Strahlungsquelle (20) und dem Spiegel (22) ein Strahlungspfad erstreckt, wobei die Strahlungsvorrichtung (10) dazu eingerichtet ist, dass von der Strahlungsquelle (20) emittierte elektromagnetische Strahlung durch den Strahlungspfad auf den Spiegel (22) auftreffen kann, und
wobei der Spiegel (22) so angeordnet ist, dass die aus dem Strahlungspfad auf den Spiegel (22) auftreffende elektromagnetische Strahlung vom Spiegel (22) so reflektiert wird, dass die reflektierte elektromagnetische Strahlung durch das Strahlungsaustrittsfenster (18) aus dem Gehäuse (12) austritt,
wobei die Strahlungsvorrichtung (10) zumindest einen Photosensor (26) umfasst,
**dadurch gekennzeichnet, dass**
- der Spiegel (22) zwischen dem Photosensor (26) und der Strahlungsquelle (20) angeordnet ist, wobei der Spiegel (22) eine durchgehende Ausnehmung (28) umfasst, wobei die Strahlungsvorrichtung (10) so ausgelegt ist, dass ein Teil der aus dem Strahlungspfad auf den Spiegel (22) auftreffenden elektromagnetischen Strahlung der Wellenlänge λ durch die durchgehende Ausnehmung (28) hindurch auf den Photosensor (26) geleitet wird,
oder
- die Strahlungsvorrichtung (10) so ausgelegt ist, dass die Position des Spiegels (22) und/oder die Ausrichtung des Spiegels (22) reversibel und zerstörungsfrei so geändert werden kann,
x) dass die aus dem Strahlungspfad auf den Spiegel (22) auftreffende elektromagnetische Strahlung so reflektiert wird, dass die reflektierte elektromagnetische Strahlung auf den Photosensor (26) geleitet wird, oder
y) dass die elektromagnetische Strahlung am Spiegel (22) vorbei auf den Photosensor (26) geleitet wird.

2. Strahlungsvorrichtung (10) nach Anspruch 1, wobei die Strahlungsquelle (20) das Intensitätsmaximum der Emission bei einer Wellenlänge λ im Bereich von 380 bis 550 nm aufweist.

3. Strahlungsvorrichtung (10) nach einem der Ansprüche 1 oder 2, wobei die Strahlungsvorrichtung (10) zumindest eine im Strahlungspfad angeordnete Linse (24) umfasst, die dazu eingerichtet ist, die von der Strahlungsquelle (20) emittierte elektromagnetische Strahlung vor dem Auftritt auf den Spiegel (22) zu beeinflussen.

4. Strahlungsvorrichtung (10) nach Anspruch 3, wobei die Strahlungsvorrichtung (10) dazu eingerichtet ist, dass der Abstand zwischen der Linse (24) und der Strahlungsquelle (20) reversibel und zerstörungsfrei veränderbar ist.

5. Strahlungsvorrichtung (10) nach einem der Ansprüche 1 bis 4, wobei der Spiegel (22) reversibel und zerstörungsfrei beweglich im Inneren des Gehäuses (12) angeordnet ist.

6. Strahlungsvorrichtung (10) nach einem der Ansprüche 1 bis 5, wobei der Photosensor (26) dazu eingerichtet ist, für auftreffende elektromagnetische Strahlung eine Intensitätsinformation zu bestimmen, wobei die Intensitätsinformation die Intensität der Strahlung und/oder einen mit der Intensität korrelierenden Parameter umfasst.

7. Strahlungsvorrichtung (10) nach Anspruch 6, wobei die Strahlungsvorrichtung (10) eine elektronische Datenverarbeitungsvorrichtung (30) zur Steuerung und/oder Regelung der Strahlungsvorrichtung (10) umfasst, wobei die elektronische Datenverarbeitungsvorrichtung (30) dazu eingerichtet ist, die Leistung der Strahlungsquelle (20) und/oder der Abstand zwischen der Strahlungsquelle und der Linse (24) in Abhängigkeit von der Intensitätsinformation zu steuern bzw. zu regeln.

8. Strahlungsvorrichtung (10) nach einem der Ansprüche 6 oder 7, wobei die elektronische Datenverarbeitungsvorrichtung (30) dazu eingerichtet ist, die Position der Linse (24) relativ zu der Strahlungsquelle (20) entlang des Strahlungspfades reversibel und zerstörungsfrei zu verändern.

9. Strahlungsvorrichtung (10) nach einem der Ansprüche 6 bis 8, wobei die elektronische Datenverarbeitungsvorrichtung (30) dazu eingerichtet ist, die Position und/oder Ausrichtung des Spiegels (22) reversibel und zerstörungsfrei zu verändern.

10. Strahlungsvorrichtung (10) nach einem der Ansprüche 1 bis 9, wobei die Strahlungsvorrichtung (10) dazu eingerichtet ist, aus dem Strahlungsaustrittsfenster (18) elektromagnetische Strahlung mit einer Intensität von 500 mW/cm² oder mehr, zu emittieren.

11. Nicht-therapeutisches Verfahren zum Betrieb einer Strahlungsvorrichtung (10) nach einem der Ansprüche 1 bis 10, umfassend die Verfahrensschritte:
a) Erzeugen von elektromagnetischer Strahlung mit der Strahlungsquelle (20), wobei die elektromagnetische Strahlung das Intensitätsmaximum bei einer Wellenlänge λ im Bereich von 350 bis 600 nm aufweist,
b) Leiten der elektromagnetischen Strahlung im Inneren des Gehäuses (12) von der Strahlungsquelle (20) zu dem Spiegel (22) durch den Strahlungspfad, und
c) Ablenken der elektromagnetischen Strahlung mit dem Spiegel (22), so dass die reflektierte elektromagnetische Strahlung durch das Strahlungsaustrittsfenster (18) aus dem Gehäuse (12) austritt.

## Claims

1. Radiation device (10) for use in activating polymerizable dental materials, comprising:
i) a housing (12) comprising:
i.a) a base portion (14), and
i.b) a head portion (16) connected to the base portion (14), wherein the housing (12) comprises a radiation emission window (18) in the head portion (16),
ii) a radiation source (20) arranged in the region of the base portion (14) in the interior of the housing (12), for emitting electromagnetic radiation, the radiation source (20) having an emission intensity maximum at a wavelength λ in the range from 350 to 600 nm, and
iii) a mirror (22) arranged in the region of the head portion (16) in the interior of the housing (12),
wherein a radiation path extends between the radiation source (20) and the mirror (22), and the radiation device (10) is configured to allow electromagnetic radiation emitted by the radiation source (20) to arrive at the mirror (22) via the radiation path, and
wherein the mirror (22) is arranged in such a manner that the electromagnetic radiation arriving at the mirror (22) via the radiation path is reflected by the mirror (22) such that the reflected electromagnetic radiation exits the housing (12) through the radiation emission window (18),
wherein the radiation device (10) comprises at least one photosensor (26), **characterized in that**
- the mirror (22) is arranged between the photosensor (26) and the radiation source (20), wherein the mirror (22) comprises a continuous recess (28), wherein the radiation device (10) is designed such that a portion of the electromagnetic radiation of wavelength λ arriving at the mirror (22) via the radiation path is directed through the continuous recess (28) onto the photosensor (26),
or
- the radiation device (10) is designed such that the position of the mirror (22) and/or the orientation of the mirror (22) can be reversibly and non-destructively changed in such a way that
x) the electromagnetic radiation arriving at the mirror (22) via the radiation path is reflected in such a way that the reflected electromagnetic radiation is directed onto the photosensor (26), or
y) the electromagnetic radiation is directed past the mirror (22) onto the photosensor (26).

2. Radiation device (10) according to claim 1, wherein the radiation source (20) has an emission intensity maximum at a wavelength λ in the range from 380 to 550 nm.

3. Radiation device (10) according to any of claims 1 or 2, wherein the radiation device (10) comprises at least one lens (24) arranged in the radiation path and configured to influence the electromagnetic radiation emitted by the radiation source (20) before it arrives at the mirror (22).

4. Radiation device (10) according to claim 3, wherein the radiation device (10) is configured so that the distance between the lens (24) and the radiation source (20) can be changed reversibly and non-destructively.

5. Radiation device (10) according to any of claims 1 to 4, wherein the mirror (22) is reversibly and non-destructively movably arranged in the interior of the housing (12).

6. Radiation device (10) according to any of claims 1 to 5, wherein the photosensor (26) is designed to determine intensity information for incoming electromagnetic radiation, wherein the intensity information comprises the intensity of the radiation and/or a parameter correlating with the intensity.

7. Radiation device (10) according to claim 6, comprising an electronic data processing device (30) for controlling and/or regulating the radiation device (10), wherein the electronic data processing device (30) is configured to control the power of the radiation source (20) and/or the distance between the radiation source and the lens (24) in accordance with the intensity information.

8. Radiation device (10) according to any of claims 6 or 7, wherein the electronic data processing device (30) is configured to reversibly and non-destructively change the position of the lens (24) relative to the radiation source (20) along the radiation path.

9. Radiation device (10) according to any of claims 6 to 8, wherein the electronic data processing device (30) is configured to reversibly and non-destructively change the position and/or orientation of the mirror (22).

10. Radiation device (10) according to any of claims 1 to 9, wherein the radiation device (10) is designed to emit electromagnetic radiation with an intensity of 500 mW/cm² or more from the radiation emission window (18).

11. Non-therapeutic method for operating a radiation device (10) according to any of claims 1 to 10, comprising the method steps of:
a) generating electromagnetic radiation using the radiation source (20), the electromagnetic radiation having an intensity maximum at a wavelength λ in the range from 350 to 600 nm,
b) guiding the electromagnetic radiation in the interior of the housing (12) from the radiation source (20) to the mirror (22) via the radiation path, and
c) deflecting the electromagnetic radiation using the mirror (22) so that the reflected electromagnetic radiation exits the housing (12) through the radiation emission window (18).

## Revendications

1. Dispositif de rayonnement (10) pour une utilisation destinée à activer des matériaux dentaires polymérisables, comprenant :
i) un boîtier (12), comprenant :
i.a) une section de base (14), et
i.b) une section supérieure (16) reliée avec la section de base (14), le boîtier (12) comprenant dans la section supérieure (16) une fenêtre de sortie de rayonnement (18),
ii) une source de rayonnement (20) agencée dans la zone de la section de base (14) à l'intérieur du boîtier (12) pour émettre un rayonnement électromagnétique, la source de rayonnement (20) présentant l'intensité maximale de l'émission à une longueur d'onde λ comprise entre 350 et 600 nm, et
iii) un miroir (22) agencé dans la zone de la section supérieure (16) à l'intérieur du boîtier (12),
un trajet de rayonnement s'étendant entre la source de rayonnement (20) et le miroir (22), le dispositif de rayonnement (10) étant conçu pour que le rayonnement électromagnétique émis par la source de rayonnement (20) puisse incider sur le miroir (22) en passant par le trajet de rayonnement, et
le miroir (22) étant agencé de telle sorte que le rayonnement électromagnétique incident sur le miroir (22) en sortant du trajet de rayonnement soit réfléchi par ledit miroir (22) pour que le rayonnement électromagnétique réfléchi sorte du boîtier (12) en passant par la fenêtre de sortie de rayonnement (18),
le dispositif de rayonnement (10) comprenant au moins un photocapteur (26), **caractérisé en ce que**
- le miroir (22) est agencé entre le photocapteur (26) et la source de rayonnement (20), ledit miroir (22) comprenant un évidement continu (28), le dispositif de rayonnement (10) étant conçu pour qu'une partie du rayonnement électromagnétique de longueur d'onde λ, qui sort du trajet de rayonnement et incide sur le miroir (22), soit dirigée sur le photocapteur (26) en passant au travers de l'évidement continu (28),
ou que
- le dispositif de rayonnement (10) est conçu pour que la position du miroir (22) et/ou la direction du miroir (22) puissent être modifiées de manière réversible et non destructive pour que
x) le rayonnement électromagnétique sortant du trajet de rayonnement et incident sur le miroir (22) soit réfléchi de telle sorte que le rayonnement électromagnétique réfléchi soit dirigé sur le photocapteur (26), ou
y) que le rayonnement électromagnétique soit dirigé sur le photocapteur (26) sans passer par le miroir (22).

2. Dispositif de rayonnement (10) selon la revendication 1, la source de rayonnement (20) présentant l'intensité maximale de l'émission à une longueur d'onde λ comprise entre 380 et 550 nm.

3. Dispositif de rayonnement (10) selon l'une quelconque des revendications 1 ou 2, ledit dispositif de rayonnement (10) comprenant au moins une lentille (24) agencée sur le trajet de rayonnement et destinée à influencer le rayonnement électromagnétique émis par la source de rayonnement (20) avant qu'il n'incide sur le miroir (22).

4. Dispositif de rayonnement (10) selon la revendication 3, ledit dispositif de rayonnement (10) étant conçu pour que la distance entre la lentille (24) et la source de rayonnement (20) puisse être modifiée de manière réversible et non destructive.

5. Dispositif de rayonnement (10) selon l'une quelconque des revendications 1 à 4, le miroir (22) étant agencé mobile dans l'intérieur du boîtier (12), et ce de manière réversible et non destructive.

6. Dispositif de rayonnement (10) selon l'une quelconque des revendications 1 à 5, le photocapteur (26) étant destiné à déterminer une information d'intensité relative à un rayonnement électromagnétique incident, ladite information d'intensité comprenant l'intensité du rayonnement et/ou un paramètre en corrélation avec l'intensité.

7. Dispositif de rayonnement (10) selon la revendication 6, ledit dispositif de rayonnement (10) comprenant un dispositif de traitement de données électronique (30) destiné à commander et/ou régler le dispositif de rayonnement (10), ledit dispositif de traitement de données électronique (30) étant conçu pour commander ou régler la puissance de la source de rayonnement (20) et/ou la distance entre la source de rayonnement et la lentille (24) en fonction de l'information d'intensité.

8. Dispositif de rayonnement (10) selon l'une quelconque des revendications 6 ou 7, le dispositif de traitement de données électronique (30) étant conçu pour modifier de manière réversible et non destructive la position de la lentille (24) par rapport à la source de rayonnement (20) le long du trajet de rayonnement.

9. Dispositif de rayonnement (10) selon l'une quelconque des revendications 6 à 8, le dispositif de traitement de données électronique (30) étant destiné à modifier de manière réversible et non destructive la position et/ou la direction du miroir (22).

10. Dispositif d'irradiation (10) selon l'une quelconque des revendications 1 à 9, ledit dispositif d'irradiation (10) étant destiné à émettre du rayonnement électromagnétique sortant de la fenêtre de sortie de rayonnement (18) et d'une intensité de 500 mW/cm² ou plus.

11. Procédé non-thérapeutique d'exploitation d'un dispositif de rayonnement (10) selon l'une quelconque des revendications 1 à 10, comprenant les étapes de procédé :
a) générer du rayonnement électromagnétique avec la source de rayonnement (20), le rayonnement électromagnétique présentant l'intensité maximale à une longueur d'onde λ comprise entre 350 et 600 nm,
b) conduire du rayonnement électromagnétique à l'intérieur du boîtier (12), de la source de rayonnement (20) au miroir (22) en passant par le trajet de rayonnement, et
c) dévier le rayonnement électromagnétique au moyen du miroir (22), de telle sorte que le rayonnement électromagnétique réfléchi sorte du boîtier (12) par la fenêtre de sortie de rayonnement (18).
